# EUROPEAN PATENT APPLICATION

(11) **EP 2 601 952 A1**
(43) Date of publication of application: **12.06.2013**
(21) Application number: 11009632.8
(22) Date of filing: 07.12.2011
(51) Int. Cl.: A61K 31/444, C07D 213/61

(54) **Novel pharmaceutically acceptable salts and cocrystals of 5-chloro-3-(4-methanesulfonylphenyl)-6'-methyl-[2,3']bipyridinyl and their therapeutic uses**

(71) Applicant: Zentiva, k.s., 102 37 Praha 10 (CZ)
(72) Inventor: Miller, Gary, Macclesfield Cheshire SK10 2DP (GB); Ridvan, Ludek, 102 00 Praha 10 (CZ); Baumgartner, Bruno, 64287 Darmstadt (DE); Nagel, Norbert, 64546 Mörfelden-Walldorf (DE)
(74) Representative: Jirotkova, Ivana

(57) **Abstract**

New crystalline forms of 5-Chloro-3-(4-methanesulfonylphenyl)-6'-methyl-[2,3']bipyridinyl in the form of stable salts and cocrystals with pharmaceutically acceptable inorganic and/or organic acids. Organic acid is selected from succinic, adipic, fumaric, benzoic, salicylic, 1-hydroxy-2-naphthoic, 3-hydroxy-2-naphthoic acid and saccharin and inorganic acid is selected from hydrochloric, hydrobromic, nitric and sulfuric acid. By the appropriate use of the salts and cocrystals described herein it is possible to modulate and ensure the consistency of the physicochemical properties of the active pharmaceutical ingredient and enable its administration in different dosage forms including for oral delivery.

## Description

### Technical Field

The present invention relates to novel solid forms of etoricoxib (I), specifically novel pharmaceutically acceptable salts and cocrystals thereof, as well as to methods of their preparation.

### Background Art

5-Chloro-3-(4-methanesulfonylphenyl)-6'-methyl-[2,3']bipyridinyl, also known as etoricoxib, of formula (I) 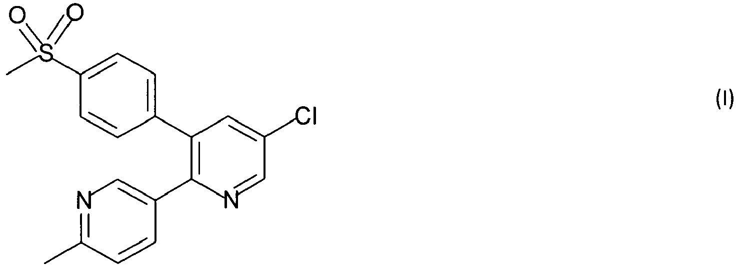 is an inhibitor of the cyclooxygenase-2 (COX-2) enzyme, a component of the arachidonic acid cascade in inflammation and is licensed as a treatment for chronic and acute pain.

Solid dosage forms are preferred for pharmaceuticals as they offer advantages in their ease of transport, storage and administration and improved patient compliance, particularly for long-term outpatient treatment. Crystalline solid forms of APIs are preferred over amorphous forms as crystalline forms are thermodynamically stable and have more predicable physical properties and better compatibility with processing equipment. The physicochemical properties of a crystalline solid are influenced by number, type and magnitude of the intermolecular interactions that determine its crystalline structure. For this reason the selection of an appropriate solid form of an API is of critical importance in ensuring that the API possesses the required physicochemical properties to be effective when administered as a medicament and also to improve its ease of manufacture and stability during storage. Key physicochemical properties include aqueous solubility and dissolution rate, melting point, chemical stability and hygroscopicity.

It is possible for materials to adopt more than one chemically identical crystalline structure, in these cases the different crystalline forms are known as polymorphs. Due to the differences in the intermolecular forces stabilizing their structures different polymorphs of the same chemical matter can exhibit different physicochemical properties and in many cases these differences can be pronounced enough to render one of the polymorphs ineffective as a medicament. For this reason the control of the polymorphic form obtained during the manufacture of the active pharmaceutical ingredient is of critical importance as the approval and licensing of a drug is specific to a particular polymorph or defined ratio of polymorphs.

The intimate relationship between crystalline structure and physical properties can be exploited to develop new solid forms of an active pharmaceutical ingredient with improved physicochemical properties by incorporation of a second molecular species into the crystalline lattice. Traditionally this has been achieved for molecules possessing acidic or basic functional groups through the formation of a crystalline salt using a pharmaceutically acceptable organic or inorganic counterion. Such counterions generally include inorganic acids such as hydrochloric, hydrobromic, sulfuric and nitric acid and organic acids such as carboxylic, dicarboxylic, hydroxy and sulfonic acids for basic API molecules and sodium, potassium, calcium, magnesium, ammonia and amines for acidic APIs. The selection of the most appropriate salt form is recognized as a critical preformulation step in the development of a novel API.

Salt formation is impossible for neutral APIs and the range of possible counterions for weakly acidic or weakly basic APIs is limited by the requirement for a difference of 3 units in the pKas of the API and counterion to reliably form a salt. Faced with these limitations, the formation of pharmaceutically acceptable cocrystals of active pharmaceutical molecules offers an alternative approach to the generation of new solid forms of the active substance. In this context a cocrystal is understood to be a binary molecular crystal containing the molecules of the API together with another molecular species in a defined stoichiometric ratio where both components are in their neutral state. The second component in the cocrystal (the component other than the active pharmaceutical ingredient) is commonly referred to as a "cocrystal former". Pharmaceutically acceptable cocrystal formers include any molecule considered acceptable as a counterion for a pharmaceutical salt or known as a pharmaceutical excipient. A widely accepted definition of a pharmaceutical cocrystal is a crystalline system containing an active pharmaceutical molecule and a cocrystal former that is a solid at ambient temperature and pressure in a defined stoichiometric ratio. This definition distinguishes cocrystals from crystalline solvates, in which case one of the components is a liquid at ambient temperature and pressure.

In common with single-component systems, different polymorphs of a crystalline molecular complex can exhibit differences in their physicochemical properties such as their aqueous solubilities and melting points. Polymorphic changes can occur as a result of changes in crystallisation conditions including the solvents and temperatures used; the presence of impurities, exposure to mechanical stress or impact or by gradual transition to a more stable form during storage. APIs that show a high degree of polymorphism are therefore challenging candidates for the reproducible manufacture of a particular polymorph or defined mixture of polymorphs.

The identification of crystalline forms may be a non-trivial process and the use of complementary techniques including X-Ray powder diffraction (XRPD), differential scanning calorimetry (DSC) and vibrational spectroscopy (for example Raman spectroscopy) is advisable to clearly and unambiguously identify the crystalline form obtained. X-ray powder diffraction is the routine method for unambiguously characterising crystalline phases and, after suitable calibration, assessing phase purity.

First synthesis of etoricoxib is described in WO 98/003484. Preparation of etoricoxib salts is mentioned (Examples 46 and 47). No solid state analytical data, like melting point or XRPD, are attached, which implies that these salts were prepared in amorphous form.

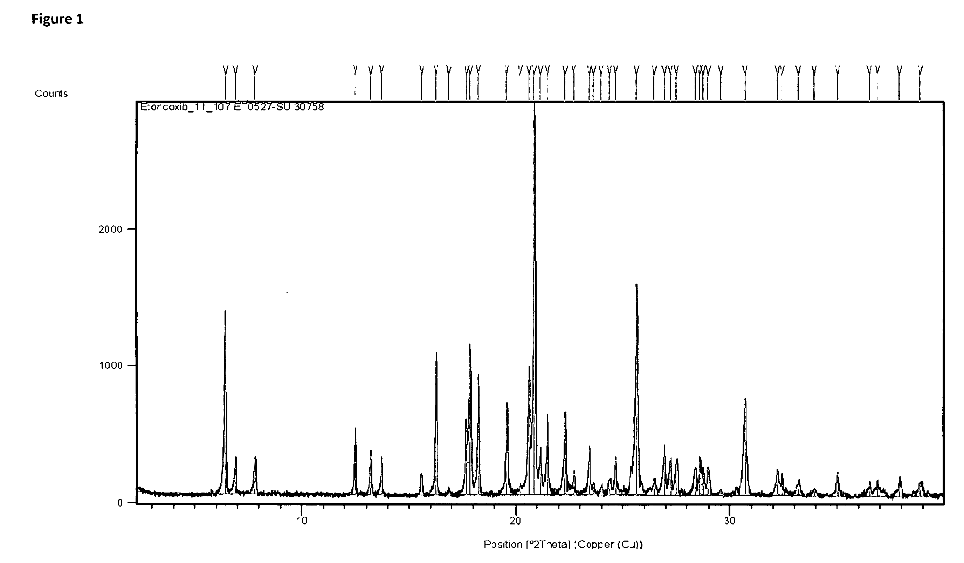

Many polymorphic forms have been described for etoricoxib (EP1248 618, WO01092230, WO 2005/085199). The instability of anhydrous etoricoxib crystalline forms I, II, III, IV and V is described in Journal of Pharmaceutical Sciences, 95, 1, 56-69 (2006). These forms convert to hemihydrate (Fig. 1).

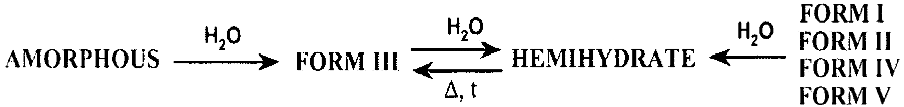

Surprisingly, no crystalline salts or co-crystals have been prepared and characterised. It is obvious that there is a need for stable etoricoxib solid forms that are suitable for the manufacture of a medicament.

### Description of the invention

1. The present invention relates to new crystalline forms of 5-Chloro-3-(4-methanesulfonylphenyl)-6'-methyl-[2,3']bipyridinyl in the form of stable salts and cocrystals with pharmaceutically acceptable inorganic and/or organic acids. An appropriate organic acid is selected from succinic, adipic, fumaric, benzoic, salicylic, 1-hydroxy-2-naphthoic, 3-hydroxy-2-naphthoic acid and saccharin and an appropriate inorganic acid is selected from hydrochloric, hydrobromic, nitric and sulfuric acid.

By the appropriate use of the salts and cocrystals described herein it is possible to modulate and ensure the consistency of the physicochemical properties of the active pharmaceutical ingredient and enable its administration in different dosage forms including for oral delivery.

In particular this invention relates to substantially pure crystalline forms of the active pharmaceutical ingredient 5-chloro-3-(4-methanesulfonylphenyl)-6'-methyl-[2,3']bipyridinyl as pharmaceutically acceptable salts and cocrystals, methods of preparing these salts and cocrystals and to the use of said salts and cocrystals in the preparation of pharmaceutical formulations for the treatment of chronic or acute pain. More particularly, these salts and cocrystals can be prepared reproducibly and have bioavailability, stability, hygroscopicity and mechanical properties that make them suitable for use in the preparation of pharmaceutical formulations that can satisfy the regulations in force governing the quality of pharmaceutical preparations. This aspect is of particular importance given the observed tendency of etoricoxib as a single-component crystalline phases to form hydrates and the difficulty in obtaining a pure polymorphic form or defined mixture of polymorphs and therefore represents a useful improvement over the currently described single-component forms.

The expression "substantially pure crystalline form" as used herein means a crystalline form characterized by XRPD that contains no more than traces of the signals relating to other crystalline forms. Preferably, the presence of such signals is equal to or below the limit of detection (LOD) of the method used and therefore, in the majority of the cases described herein, the expression "substantially pure crystalline form" means a crystalline form with a purity of at least 90%.
2. The preferred embodiment of the invention is the succinic acid salt of 5-chloro-3-(4-methanesulfonylphenyl)-6'-methyl-[2,3']bipyridinyl, having the empirical formula (C15 H17 N 02, C6 H8 07), characterised by the XRPD pattern with major peaks at the following 2-theta values measured using CuKα radiation: 6.4, 16.3, 17.9, 18.2, 19.6, 20.9, 22.3, 25.6 and 30.7° ± 0.5°.

The XRPD pattern is given in Figure 1 and comprising the main peaks listed in Table 1.

**Table 1 Table of diffraction peaks for the succinic acid salt of etoricoxib.**

| Position (2θ) | d-spacing [Å]=0.1nm | Rel. Int. (%) |
|---|---|---|
| 6.41 | 13.788 | 46.0 |
| 6.88 | 12.839 | 7.9 |
| 7.80 | 11.328 | 9.8 |
| 12.48 | 7.087 | 17.5 |
| 13.21 | 6.696 | 10.4 |
| 13,72 | 6.448 | 10.4 |
| 16.27 | 5.443 | 36.8 |
| 17.87 | 4.960 | 38.1 |
| 18.24 | 4.859 | 31.1 |
| 19.58 | 4.529 | 23.9 |
| 20.88 | 4.251 | 100.0 |
| 21.48 | 4.133 | 18.4 |
| 22.31 | 3.981 | 20.9 |
| 23.43 | 3.794 | 12.6 |
| 25.63 | 3.472 | 55.8 |
| 26.93 | 3.308 | 13.0 |
| 28.61 | 3.117 | 9.1 |
| 30.71 | 2.909 | 24.3 |
| 32.22 | 2.776 | 6.4 |
| 35.03 | 2.559 | 5.9 |

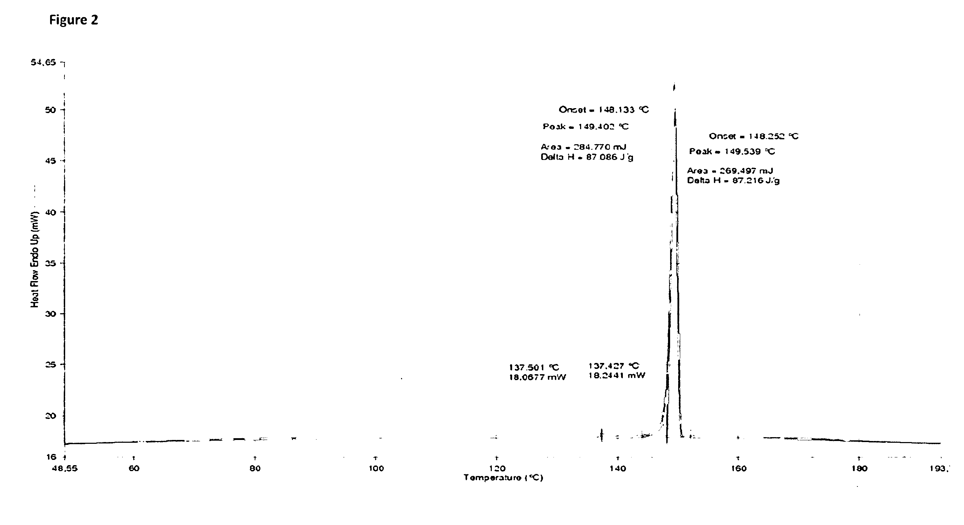

This succinic acid salt of 5-chloro-3-(4-methanesulfonylphenyl)-6'-methyl-[2,3']bipyridinyl is further characterized by the differential scanning calorimetry (DSC) thermograph shown in Figure 2, which shows an endothermic event corresponding to a melt with an onset temperature of approximately 148 °C. The melting point is in the range 148 - 150°C. The melting point is therefore more than 10 °C higher than the any of the Forms I - V polymorphs of 5-chloro-3-(4-methanesulfonylphenyl)-6'-methyl-[2,3']bipyridinyl. The melting point of a drug substance can affect its compatibility with production and formulation equipment, with lower melting solids being more likely to melt onto equipment during routine processes such as milling and grinding. Therefore, a higher melting form of a drug substance may offer advantages with respect to the ease of manufacturing and formulation of the drug.

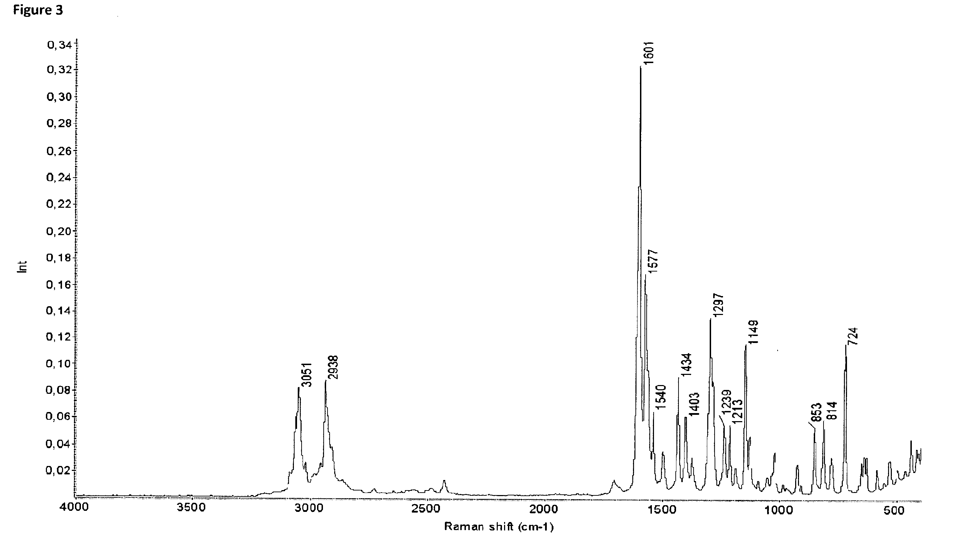

This succinic acid salt of 5-chloro-3-(4-methanesulfonylphenyl)-6'-methyl-[2,3']bipyridinyl is also characterized by the Raman spectrum given in Figure 3, which shows characteristic absorptions at approximately 724, 1149, 1297, 1577, 1601, 2938 and 3051 cm⁻¹.

In the manufacture of a solid oral dosage form it is of critical importance that the drug is manufactured and administered as a defined and consistent crystalline form (ICH, Q6A: Test Procedures and Acceptance Criteria for New Drug Products) in order to ensure that the bioavailability, stability and mechanical properties of the drug remain consistent throughout manufacture.

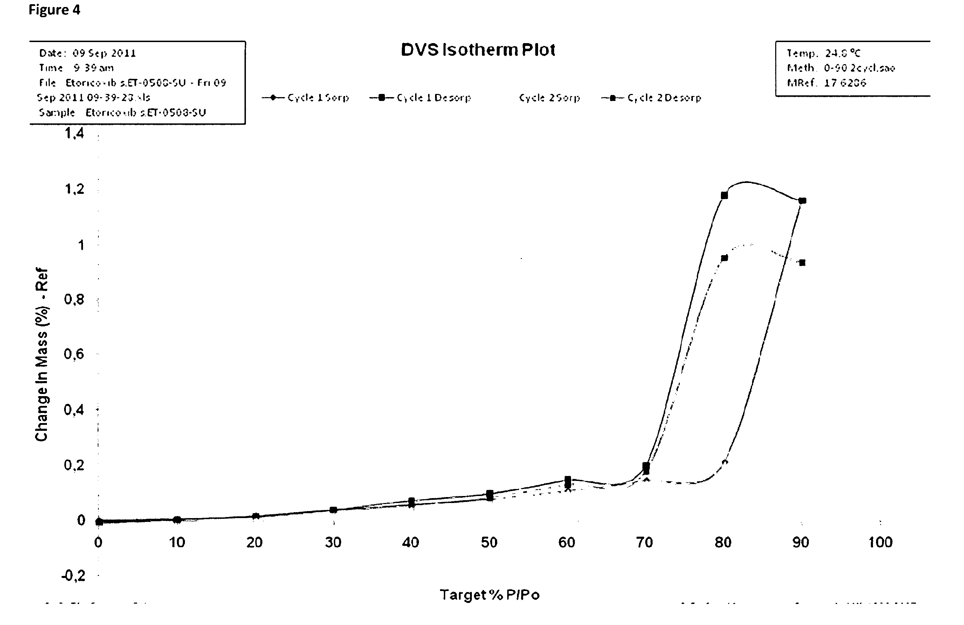

The crystallisation of the succinic acid salt of 5-chloro-3-(4-methanesulfonylphenyl)-6'-methyl-[2,3']bipyridinyl as described in Examples 1 and 2 and its lack of observed polymorphic changes when maturated in various solvents as described in Example 6 demonstrates its propensity to crystallise as a consistent and pure crystalline phase. Additionally, the high melting point and thermal stability of the cocrystal enables drying procedures to be carried out with minimal precautions. Finally, the obtained crystalline form is relatively non-hygroscopic and stable with respect to hydrate formation as demonstrated by the DVS isotherm plot shown in Figure 4.
3. A second preferred embodiment of the invention is the adipic acid salt of 5-chloro-3-(4-methanesulfonylphenyl)-6'-methyl-[2,3']bipyridinyl, which has the empirical formula (C15 H17 N 02, C6 H6 S 03) and is characterized by the XRPD pattern characterised by an XRPD pattern with major peaks at the following 2-theta values measured using CuKα radiation: 6.0, 7.5, 14.0, 17.2, 17.5, 19.7, 20.2, 21.0, 21.4, 24.4 and 28.8° ± 0.5°.

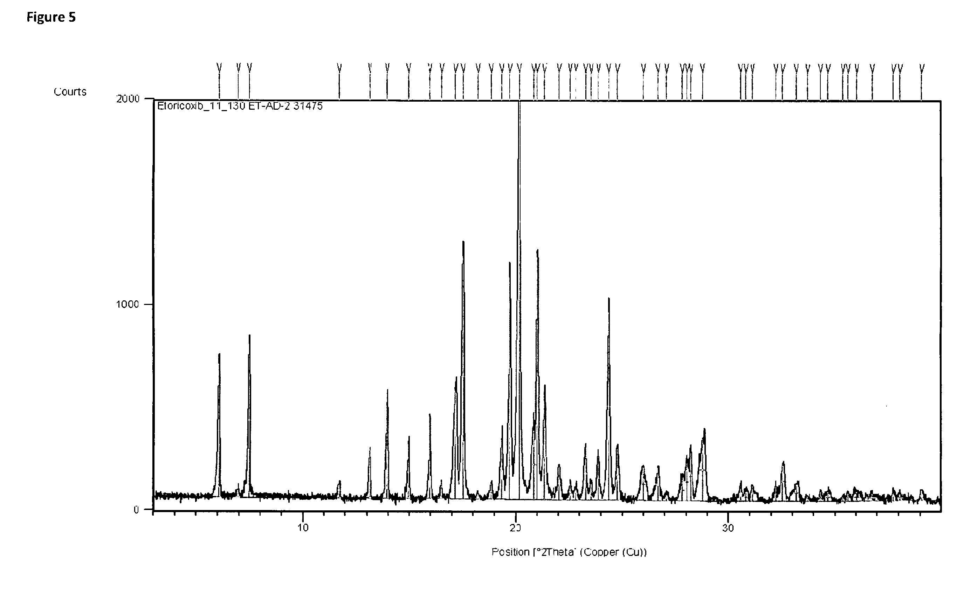

The f XPRD pattern is given in Figure 5 with the main peaks listed in Table 2.

**Table 2 Table of diffraction peaks for the adipic acid salt of 5-chloro-3-(4-methanesulfonylphenyl)-6'-methyl-[2,3']bipyridinyl.**

| Position (2θ) | d-spacing [Å] =0.1nm | Rel. Int. (%) |
|---|---|---|
| 6.04 | 14.617 | 29.7 |
| 7.46 | 11.838 | 34.4 |
| 13.14 | 6.734 | 10.7 |
| 13.96 | 6.339 | 20.7 |
| 14.98 | 5.911 | 12.0 |
| 15.97 | 5.545 | 18.1 |
| 17.15 | 5.166 | 24.2 |
| 17.53 | 5.055 | 54.6 |
| 19.73 | 4.497 | 50.1 |
| 20.17 | 4.399 | 100.0 |
| 21.03 | 4.222 | 54.8 |
| 21.36 | 4.157 | 24.6 |
| 23.27 | 3.819 | 11.7 |
| 24.38 | 3.648 | 42.3 |
| 26.00 | 3.425 | 7.4 |
| 26.68 | 3.339 | 7.3 |
| 28.77 | 3.100 | 14.0 |
| 32.57 | 2.747 | 8.9 |

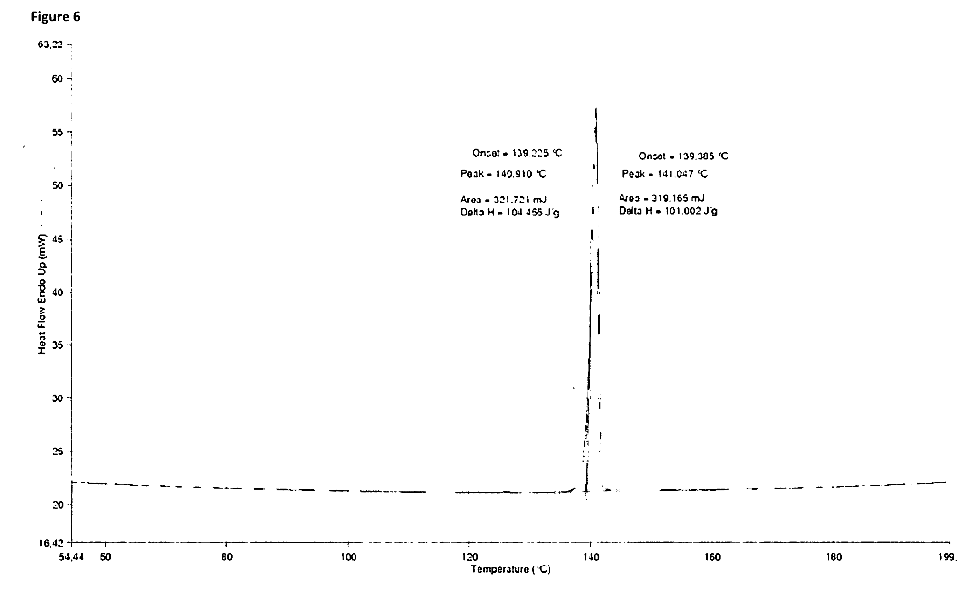

This adipic acid salt of 5-chloro-3-(4-methanesulfonylphenyl)-6'-methyl-[2,3']bipyridinyl is further characterized by the differential scanning calorimetry (DSC) thermograph shown in Figure 6, which shows an endothermic event corresponding to a melt with an onset temperature of approximately 139 °C. The melting point is in the range of 139-142°C.

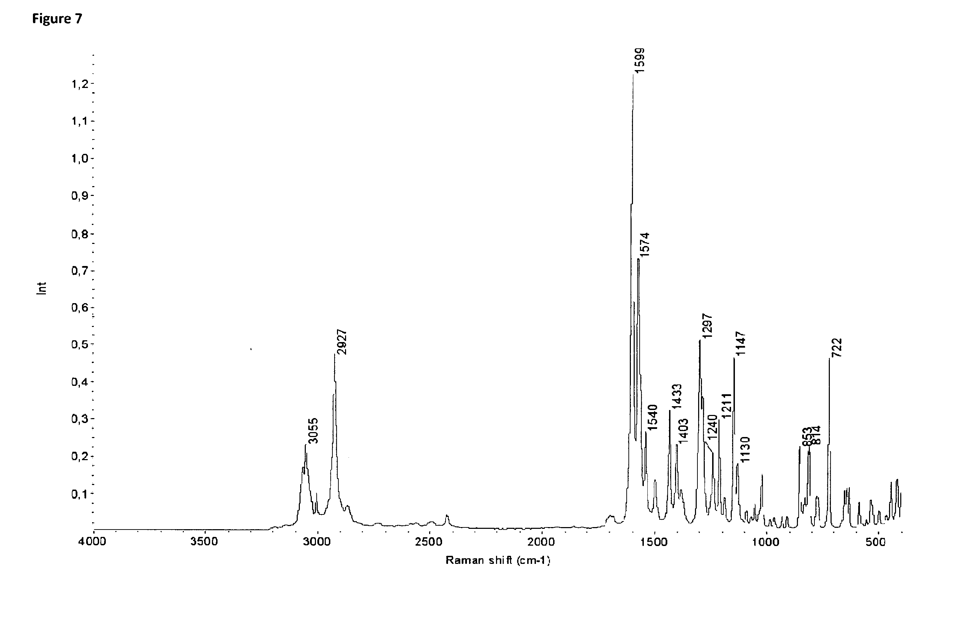

This adipic acid salt of 5-chloro-3-(4-methanesulfonylphenyl)-6'-methyl-[2,3']bipyridinyl is also characterized by the Raman spectrum given in Figure 7, which shows characteristic absorptions at approximately 3073, 1585, 1163, 1130, 1025, 997, 727, 615 and 320 cm⁻¹.

Another embodiment of the invention is the fumaric acid salt of 5-chloro-3-(4-methanesulfonylphenyl)-6'-methyl-[2,3']bipyridinyl, having the empirical formula (C₁₅ H₁₇ N O₂, C₄ H₄ O₄) and characterized by an XRPD pattern with major peaks at the following 2-theta values measured using CuKα radiation: 11.1, 12.8, 15.7, 16.2, 17.5, 18.0, 20.8, 21.6, 22.3, 23.2, 24.0, 25.6, 26.4 and 26.8° ± 0.5°.

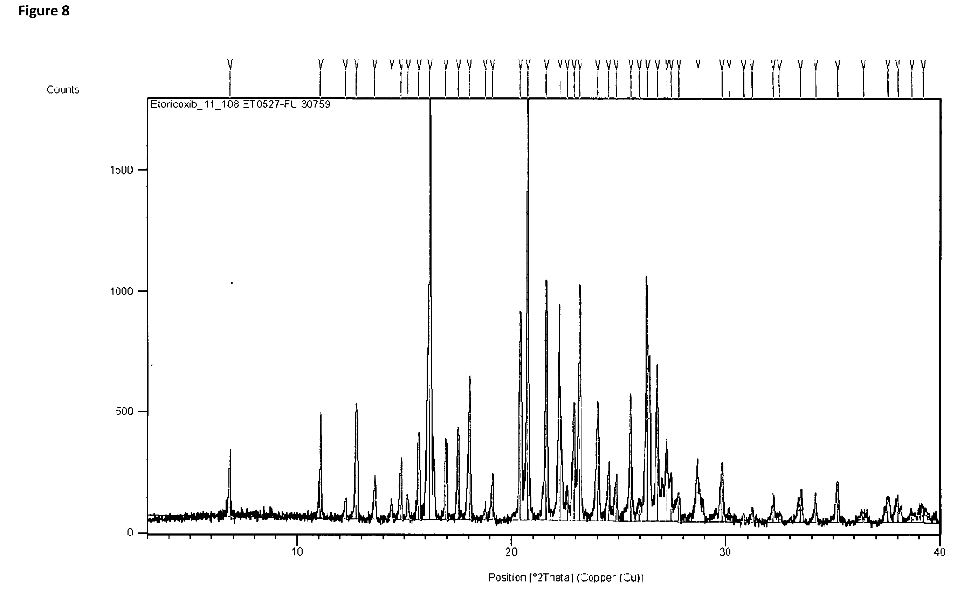

The XRPD pattern is given in Figure 8 with the main peaks listed in Table 3. This crystalline form is hereafter referred to as Form A of the fumaric acid salt.

**Table 3 Table of diffraction peaks for Form A of the fumaric acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 6.84 | 12.916 | 14.2 |
| 11.08 | 7.976 | 25.7 |
| 12.75 | 6.940 | 28.3 |
| 14.82 | 5.972 | 14.3 |
| 15.67 | 5.651 | 20.2 |
| 16.18 | 5.473 | 85.8 |
| 17.51 | 5.060 | 22.6 |
| 18.04 | 4.913 | 34.9 |
| 20.76 | 4.276 | 100.0 |
| 21.62 | 4.108 | 57.8 |
| 22.25 | 3.993 | 50.3 |
| 23.17 | 3.835 | 55.6 |
| 24.02 | 3.702 | 27.7 |
| 25.56 | 3.483 | 27.5 |
| 26.36 | 3.379 | 50.3 |
| 26.81 | 3.323 | 31.7 |
| 28.68 | 3.110 | 14.2 |
| 35.20 | 2.548 | 9.7 |

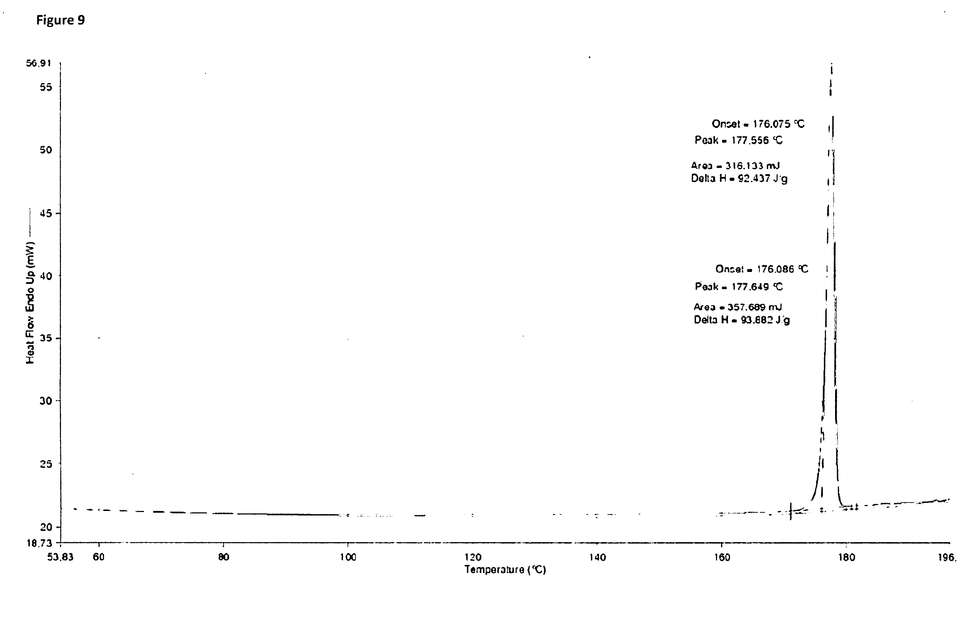

This fumaric acid salt of 5-chloro-3-(4-methanesulfonylphenyl)-6'-methyl-[2,3']bipyridinyl is further characterized by the differential scanning calorimetry (DSC) thermograph shown in Figure 9, which shows an endothermic event corresponding to a melt with an onset temperature of approximately 64 °C. The melting point is in the range of 176 to 178 °C.

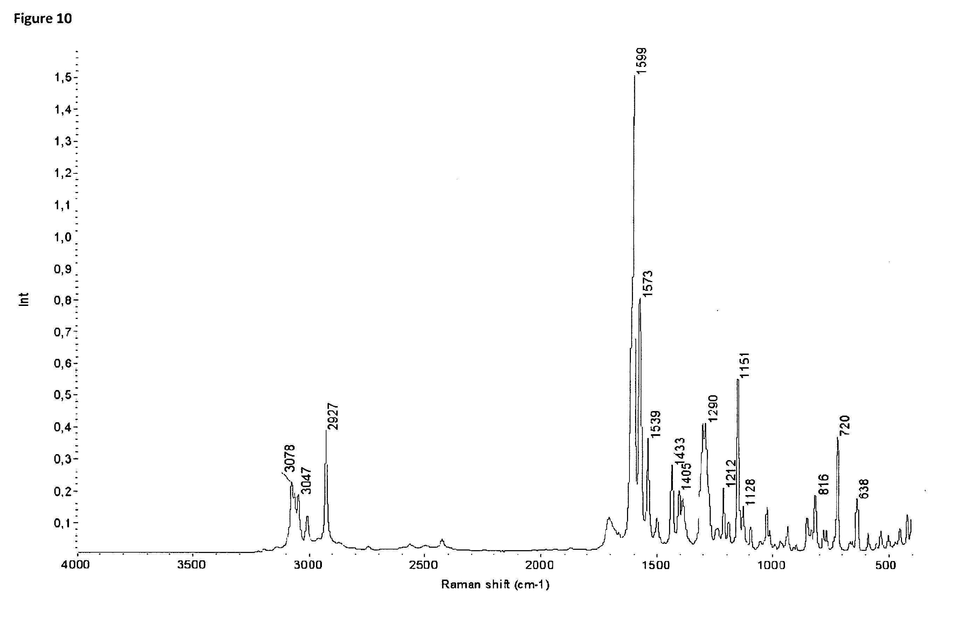

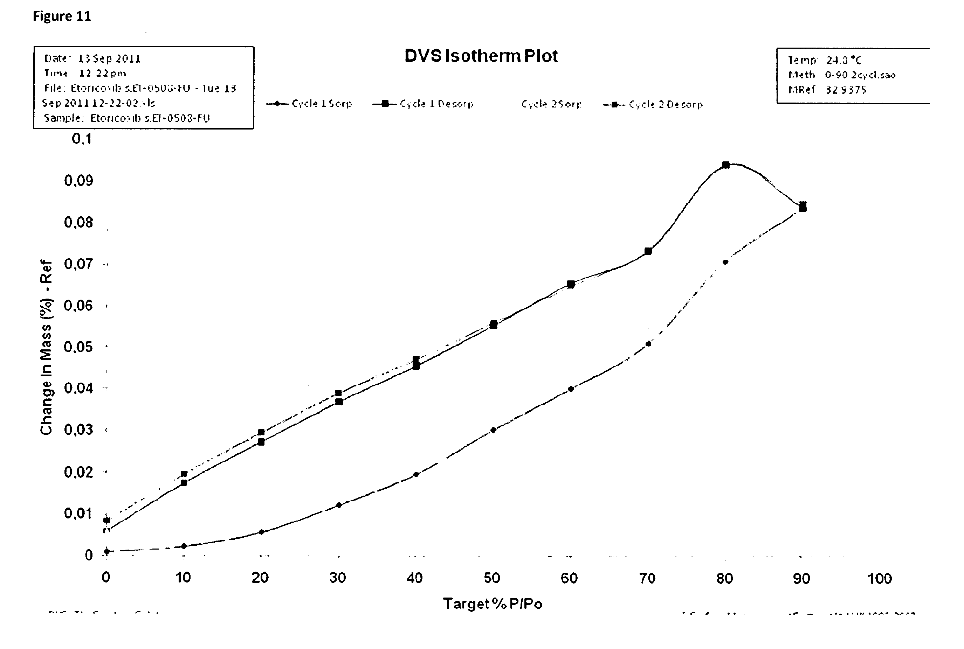

This fumaric acid salt of 5-chloro-3-(4-methanesulfonylphenyl)-6'-methyl-[2,3']bipyridinyl is also characterized by the Raman spectrum given in Figure 10, which shows characteristic absorptions at 2989, 2945, 1675, 1634, 1445, 1422, 1378, 1350, 1309, 1221, 1186, 1098, 1033, 964, 912, 883, 794, 661, 611, 534, 440, 401, 345 and 267 cm⁻¹ In common with the succinic acid salt, Form A of the fumaric acid salt is relatively non-hygroscopic as shown by the DVS isotherm plot in Figure 11.

A second crystalline form of the fumaric acid salt of 5-chloro-3-(4-methanesulfonylphenyl)-6'-methyl-[2,3']bipyridinyl has been observed. This polymorph is hereafter referred to as Form B of the fumaric acid salt. Form B can be generated by slurrying Form A of the fumaric acid salt in an aliphatic alcohol or cyclic ether at a temperature of 30 °C for 4 weeks. It is characterized by an XRPD pattern with major peaks at the following 2-theta values measured using CuKα radiation: 6.39, 16.2, 18.3, 20.9, 24.6, 25.8, 31.0° ± 0.5°.

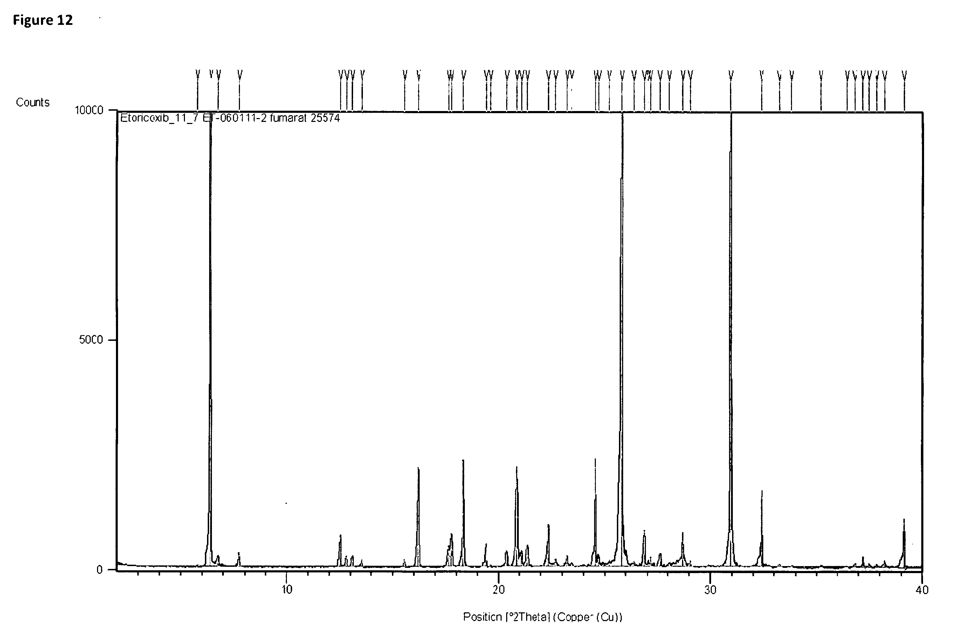

The XRPD pattern is given in Figure 12 with the main peaks listed in Table 4.

**Table 4 Table of diffraction peaks for Form B of the fumaric acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 6.39 | 13.820 | 93.8 |
| 12.50 | 7.076 | 3.2 |
| 16.20 | 5.466 | 10.6 |
| 18.34 | 4.833 | 11.2 |
| 20.87 | 4.254 | 10.6 |
| 22.32 | 3.979 | 4.1 |
| 24.57 | 3.620 | 10.5 |
| 25.80 | 3.451 | 100.0 |
| 26.84 | 3.319 | 3.7 |
| 30.95 | 2.887 | 49.7 |
| 32.40 | 2.761 | 7.4 |
| 39.13 | 2.300 | 4.8 |

Another embodiment of the invention is the benzoic acid salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 9.6, 15.4, 19.1, 20.6, 22.0, 22.3 and 27.9° ± 0.5°.

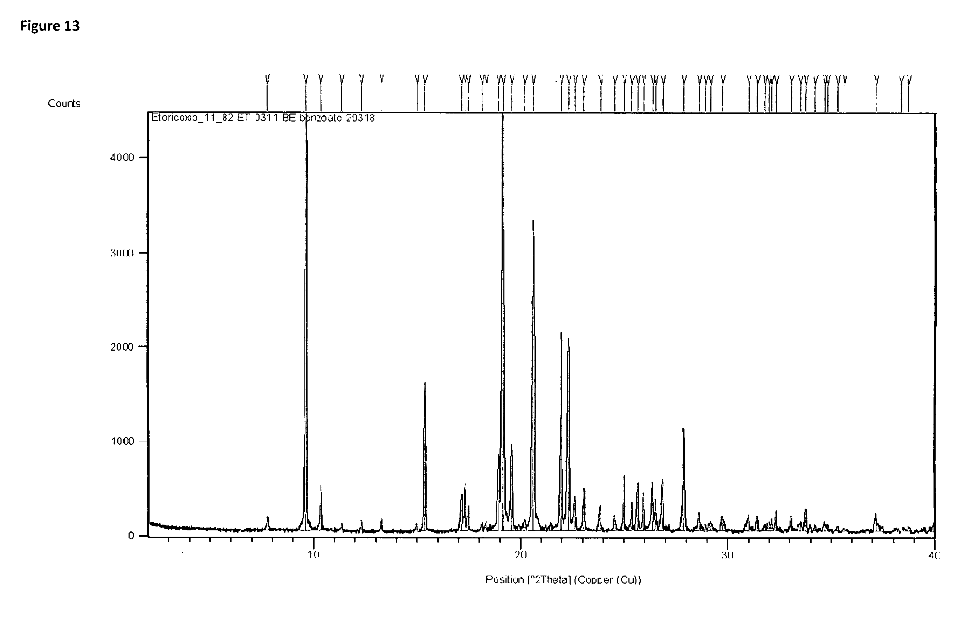

The XRPD pattern is given in Figure 13 with the main peaks listed in Table 5.

**Table 5 Table of diffraction peaks for the benzoic acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 9.62 | 9.189 | 100 |
| 10.35 | 8.541 | 8.2 |
| 15.38 | 5.756 | 24.5 |
| 17.30 | 5.121 | 8.2 |
| 19.14 | 4.633 | 76.7 |
| 20.62 | 4.304 | 54.3 |
| 21.95 | 4.045 | 34.6 |
| 22.30 | 3.984 | 33.5 |
| 23.07 | 3.851 | 7.5 |
| 25.00 | 3.559 | 9.5 |
| 25.65 | 3.471 | 8.2 |
| 26.35 | 3.380 | 8.3 |
| 26.84 | 3.319 | 9.1 |
| 27.88 | 3.198 | 17.8 |
| 32.35 | 2.765 | 3.4 |

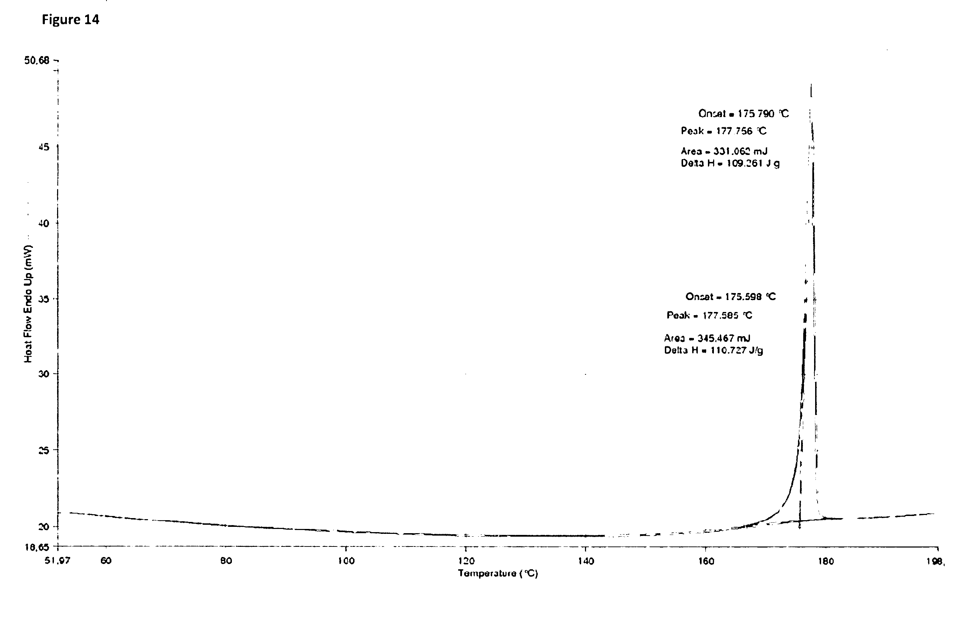

This benzoic acid salt of 5-chloro-3-(4-methanesulfonylphenyl)-6'-methyl-[2,3']bipyridinyl is further characterized by the differential scanning calorimetry (DSC) thermograph shown in Figure 14, which shows an endothermic event corresponding to a melt with an onset temperature of approximately 175 °C. The melting point is in the range of 175-9°C.

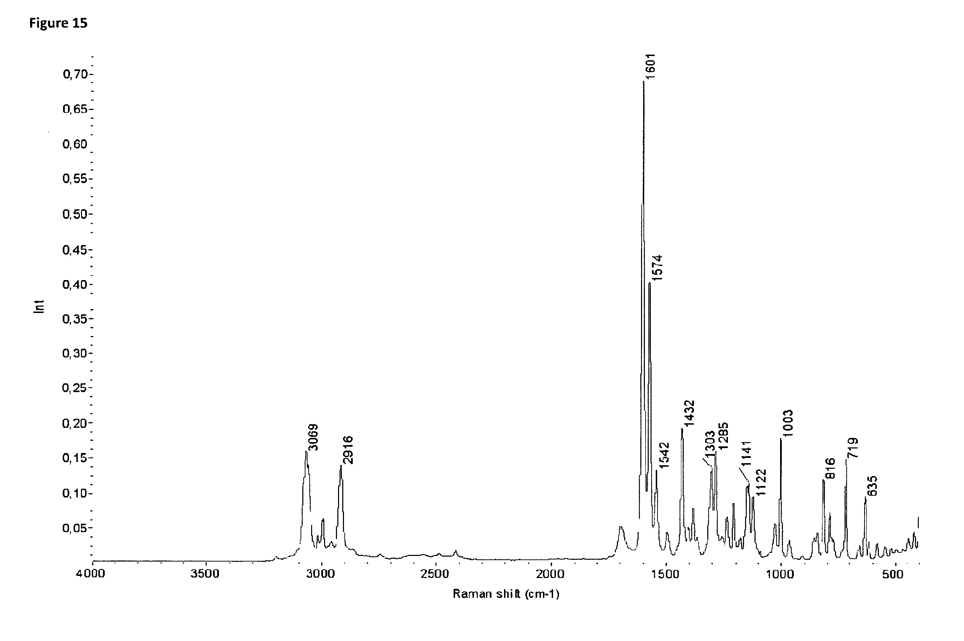

This benzoic acid salt of 5-chloro-3-(4-methanesulfonylphenyl)-6'-methyl-[2,3']bipyridinyl is also characterized by the Raman spectrum given in Figure 15, which shows characteristic absorptions at 2989, 2945, 1675, 1634, 1445, 1422, 1378, 1350, 1309, 1221, 1186, 1098, 1033, 964, 912, 883, 794, 661, 611, 534, 440, 401, 345 and 267 cm⁻¹.

Another embodiment of the invention is the salicylic acid salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 8.9, 16.8, 17.1, 17.8, 20.4, 24.4, 26.8, 27.2° ± 0.5°.

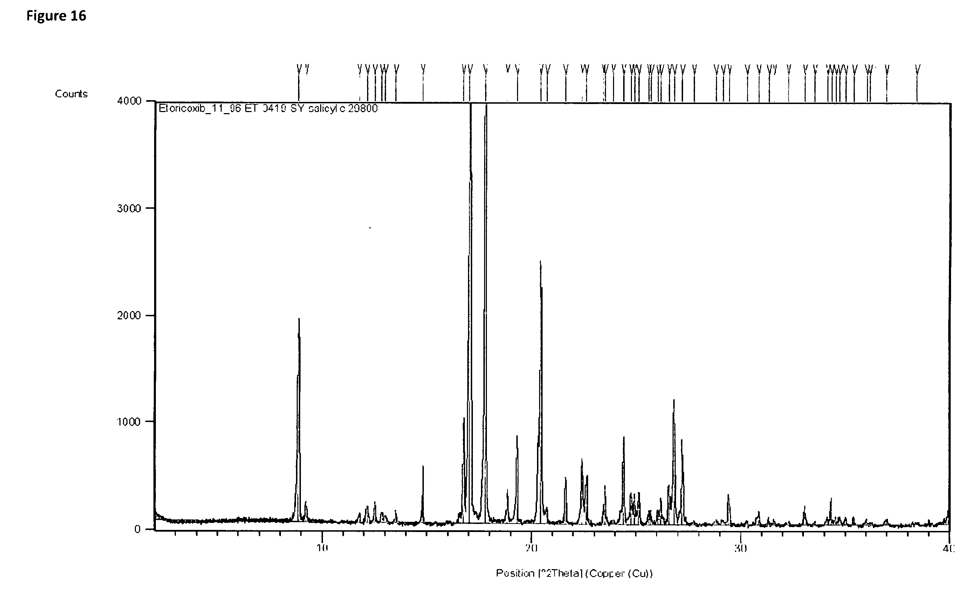

The XRPD pattern is given in Figure 16 with the main peaks listed in Table 6.

**Table 6 Table of diffraction peaks for the salicylic acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 8.88 | 9.956 | 37.4 |
| 12.52 | 7.067 | 4.0 |
| 14.81 | 5.976 | 11.5 |
| 16.75 | 5.289 | 18.8 |
| 17.06 | 5.192 | 81.4 |
| 17.79 | 4.981 | 100.0 |
| 19.30 | 4.596 | 15.2 |
| 20.44 | 4.342 | 46.2 |
| 21.63 | 4.106 | 9.6 |
| 22.41 | 3.964 | 11.9 |
| 24.40 | 3.645 | 17.2 |
| 26.82 | 3.322 | 23.5 |
| 27.22 | 3.274 | 16.1 |
| 29.43 | 3.033 | 5.6 |
| 33.08 | 2.706 | 3.3 |
| 34.34 | 2.609 | 5.7 |

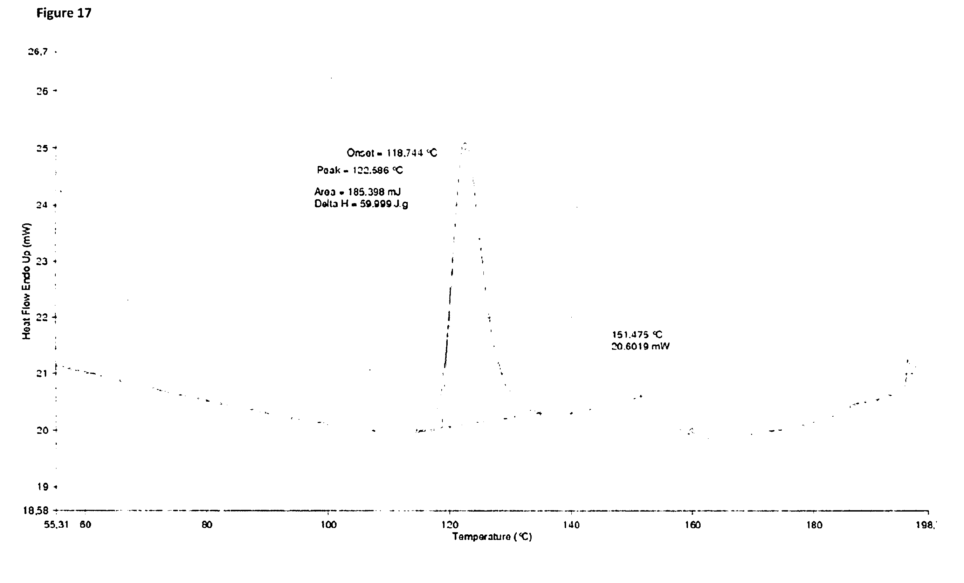

This salicylic acid salt of 5-chloro-3-(4-methanesulfonylphenyl)-6'-methyl-[2,3']bipyridinyl is further characterized by the differential scanning calorimetry (DSC) thermograph shown in Figure 17, which shows an endothermic event corresponding to a melt with an onset temperature of approximately 175 °C. The melting point is in the range of 118-124°C.

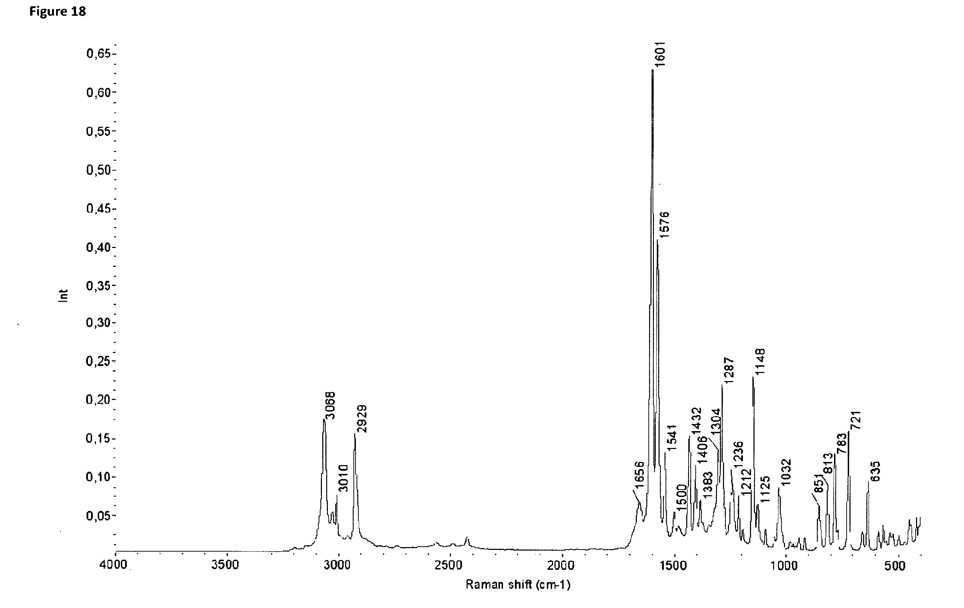

This salicylic acid salt of 5-chloro-3-(4-methanesulfonylphenyl)-6'-methyl-[2,3']bipyridinyl is also characterized by the Raman spectrum given in Figure 18, which shows characteristic absorptions at 3068, 2929, 1656, 1601, 1576,1287 and 1148 cm⁻¹.

Another embodiment of the invention is the 1-hydroxy-2-naphthoic acid salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα adiation: 7.4, 14.9, 15.2, 16.7, 18.8, 21.5, 21.8, 22.8, 24.0 and 29.6° ± 0.5°.

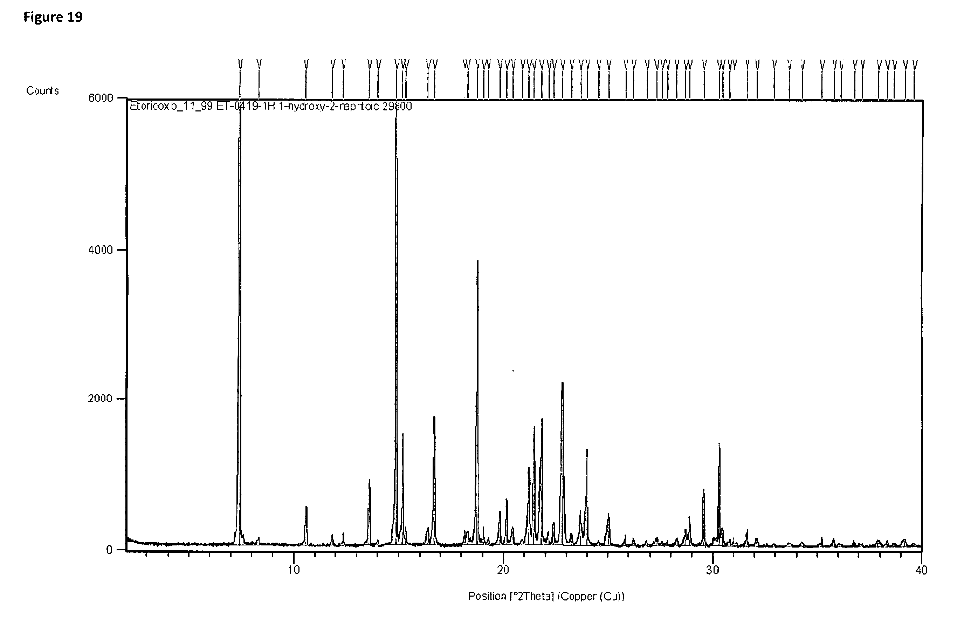

The XRPD pattern is given in Figure 19 with the main peaks listed in Table 7.

**Table 7 Table of diffraction peaks for the 1-hydroxy-2-naphthoic acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 7.40 | 11.933 | 100 |
| 10.59 | 8.351 | 6.5 |
| 13.59 | 6.510 | 12.0 |
| 14.87 | 5.952 | 88.6 |
| 15.21 | 5.822 | 20.4 |
| 16.71 | 5.302 | 21.5 |
| 18.76 | 4.727 | 51.0 |
| 20.18 | 4.396 | 8.7 |
| 21.21 | 4.186 | 13.7 |
| 21.48 | 4.133 | 21.8 |
| 21.81 | 4.073 | 23.3 |
| 22.82 | 3.894 | 28.4 |
| 23.99 | 3.706 | 17.3 |
| 25.02 | 3.556 | 6.3 |
| 28.89 | 3.088 | 5.3 |
| 29.57 | 3.019 | 10.7 |
| 30.47 | 2.931 | 3.3 |
| 31.64 | 2.825 | 3.2 |

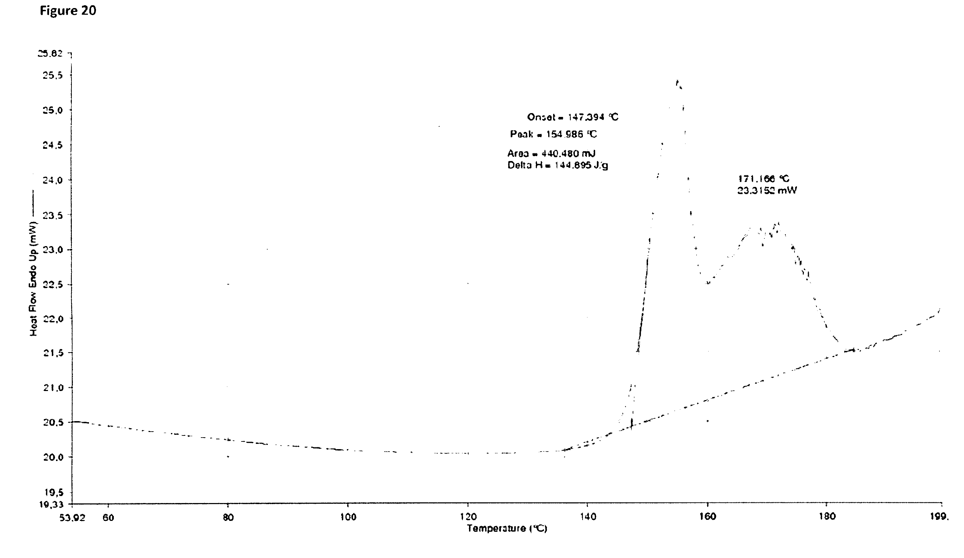

This 1-hydroxy-2-naphthoic acid salt of 5-chloro-3-(4-methanesulfonylphenyl)-6'-methyl-[2,3']bipyridinyl is further characterized by the differential scanning calorimetry (DSC) thermograph shown in Figure 20, which shows an endothermic event corresponding to a melt with an onset temperature of approximately 160 °C. The melting point is in the range 147 to 160°.

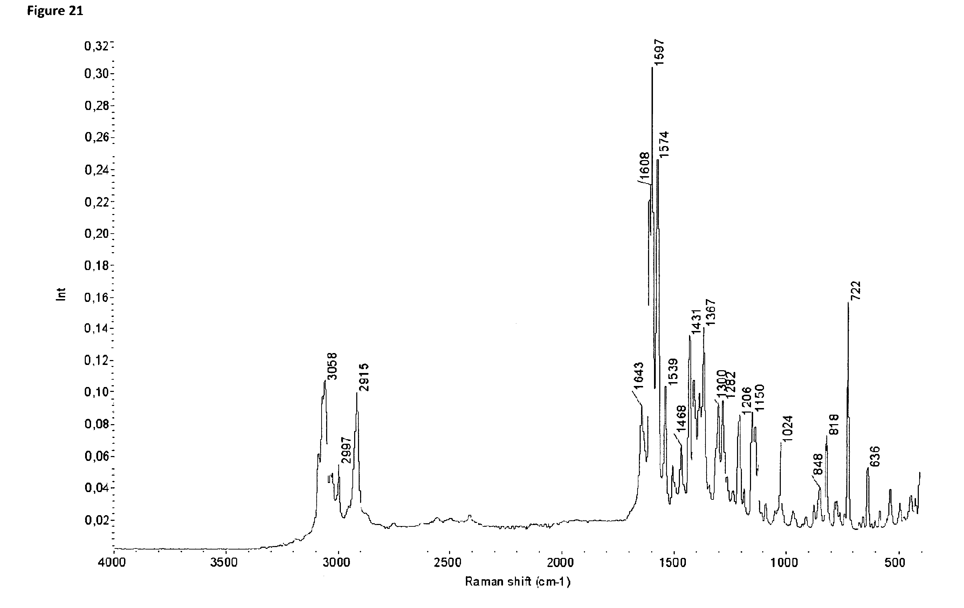

This 1-hydroxy-2-naphthoic acid salt of 5-chloro-3-(4-methanesulfonylphenyl)-6'-methyl-[2,3']bipyridinyl is also characterized by the Raman spectrum given in Figure 21, which shows characteristic absorptions at 3058, 2915, 1643, 1597, 1574, 1431 and 1367 cm⁻¹.

Another embodiment of the invention is the 3-hydroxy-2-naphthoic acid salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 6.7, 10.1, 15.3, 15.5, 17.4, 19.0, 20.1, 20.5, 21.3, 24.6 and 27.3° ± 0.5°.

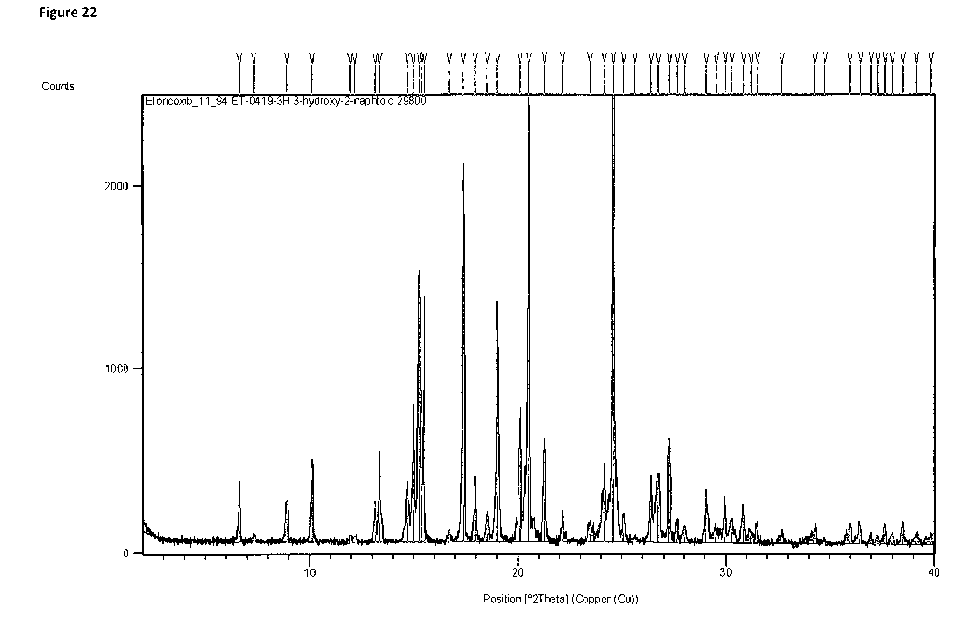

The XRPD pattern is given in Figure 22 with the main peaks listed in Table 8.

**Table 8 Table of diffraction peaks for the 3-hydroxy-2-naphthoic acid salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 6.65 | 13.280 | 11.0 |
| 8.92 | 9.907 | 8.4 |
| 10.13 | 8.728 | 14.5 |
| 15.26 | 5.800 | 51.5 |
| 15.51 | 5.709 | 47.6 |
| 17.40 | 5.092 | 69.9 |
| 19.03 | 4.659 | 42.1 |
| 20.11 | 4.413 | 21.8 |
| 20.52 | 4.324 | 92.3 |
| 21.28 | 4.172 | 17.5 |
| 24.60 | 3.615 | 100.0 |
| 26.74 | 3.332 | 13.0 |
| 27.27 | 3.268 | 20.5 |
| 29.05 | 3.071 | 9.6 |
| 29.96 | 2.980 | 8.2 |
| 30.84 | 2.897 | 6.8 |

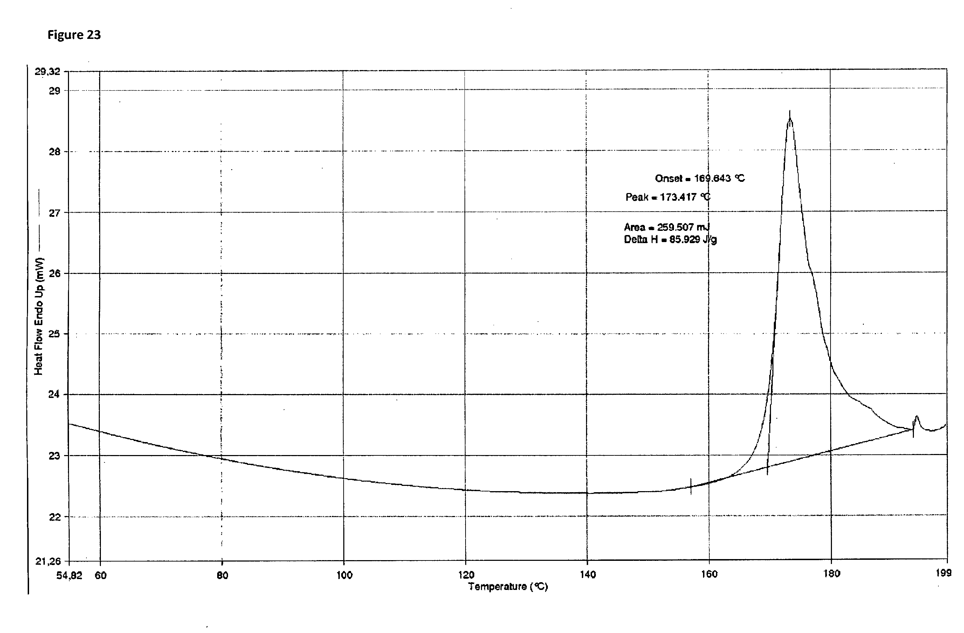

This 3-hydroxy-2-naphthoic acid salt of 5-chloro-3-(4-methanesulfonylphenyl)-6'-methyl-[2,3']bipyridinyl is further characterized by the differential scanning calorimetry (DSC) thermograph shown in Figure 23, which shows an endothermic event corresponding to a melt with an onset temperature of approximately 194 °C. The melting point is in the range 169 to 180 °C.

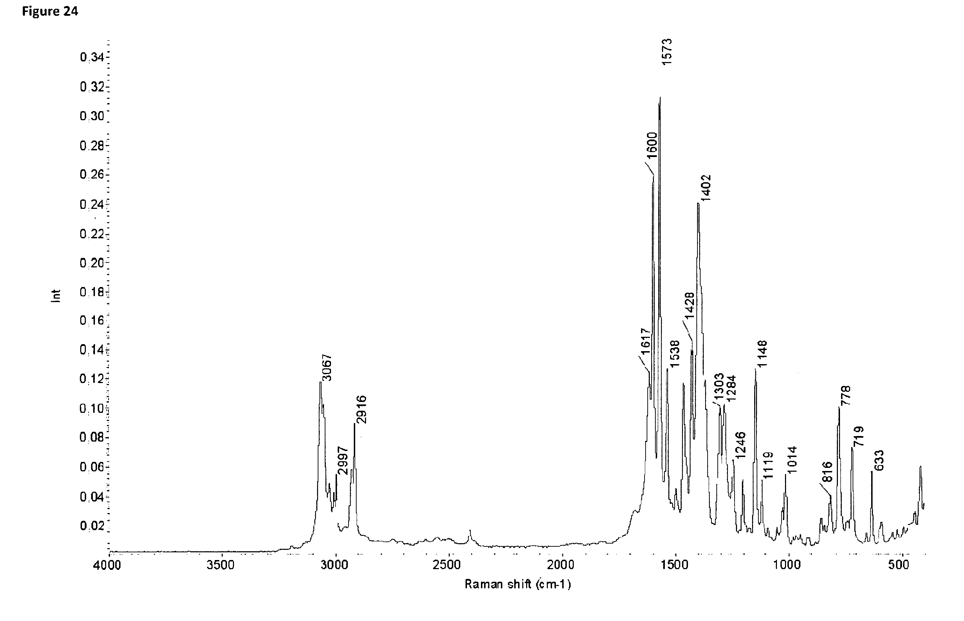

This 3-hydroxy-2-naphthoic acid salt of 5-chloro-3-(4-methanesulfonylphenyl)-6'-methyl-[2,3']bipyridinyl is also characterized by the Raman spectrum given in Figure 24, which shows characteristic absorptions at 3067, 2916, 1617, 1600, 1573, 1402, 1148 cm⁻¹.

Another embodiment of the invention is the saccharin salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 6.1, 12.2, 12.7, 15.3, 16.5, 17.7, 18.4, 19.7, 20.6, 20.9, 21.4, 22.4, 23.5, 24.0, 24.6, 25.6, 26.0, 26.4, 29.1° ± 0.5°.

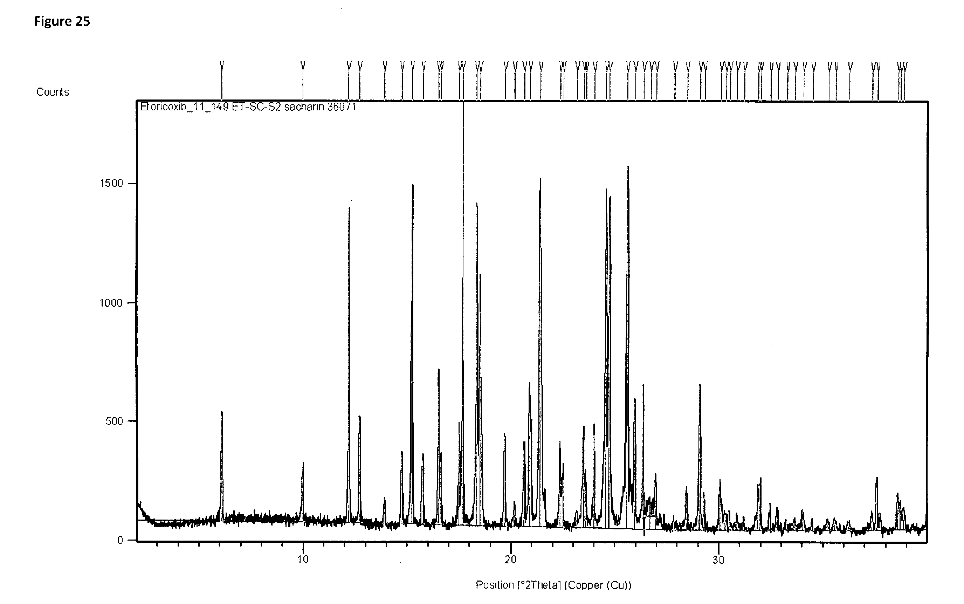

The XRPD pattern is given in Figure 25 with the main peaks listed in Table 9.

**Table 9 Table of diffraction peaks for the saccharin salt of 5-chloro-3-(4-methanesulfonylphenyl)-6'-methyl-[2,3']bipyridinyl.**

| Position (2θ) | d-spacing [Å]=0.1nm | Rel. Int. (%) |
|---|---|---|
| 6.10 | 14.476 | 26.3 |
| 9.99 | 8.851 | 14.1 |
| 12.21 | 7.240 | 76.1 |
| 12.71 | 6.958 | 26.3 |
| 15.25 | 5.807 | 70.9 |
| 16.53 | 5.358 | 34.4 |
| 17.69 | 5.011 | 100.0 |
| 18.36 | 4.827 | 80.9 |
| 19.71 | 4.501 | 22.5 |
| 20.64 | 4.300 | 19.3 |
| 20.93 | 4.241 | 34.5 |
| 21.40 | 4.148 | 80.1 |
| 22.37 | 3.971 | 21.4 |
| 23.50 | 3.783 | 22.0 |
| 24.01 | 3.704 | 23.2 |
| 24.57 | 3.620 | 76.7 |
| 25.59 | 3.478 | 86.6 |
| 25.96 | 3.430 | 31.5 |
| 26.36 | 3.378 | 30.5 |
| 28.45 | 3.135 | 10.7 |
| 29.09 | 3.068 | 36.0 |
| 30.07 | 2.969 | 11.3 |
| 31.99 | 2.795 | 11.2 |
| 38.59 | 2.331 | 8.3 |

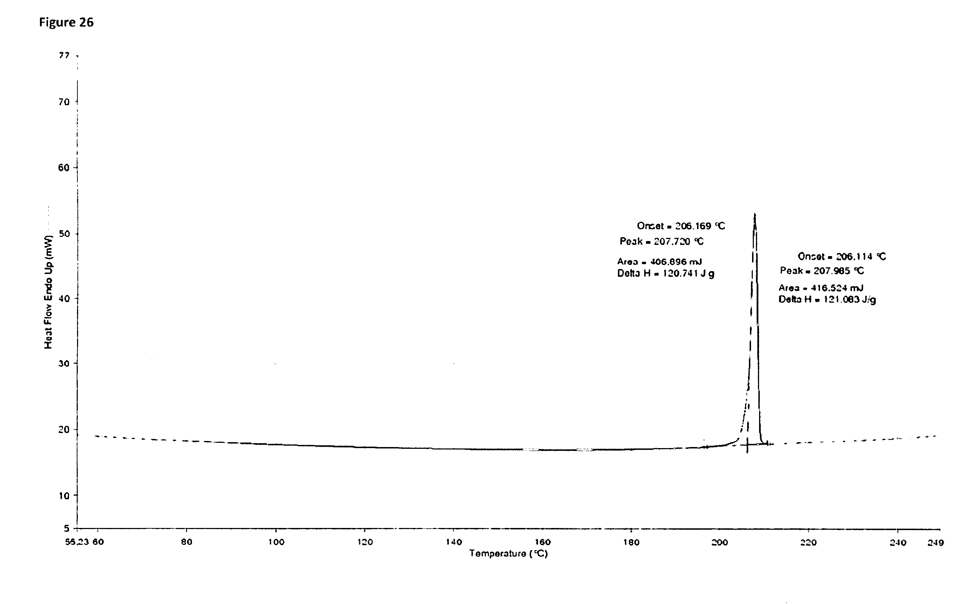

This saccharin salt of 5-chloro-3-(4-methanesulfonylphenyl)-6'-methyl-[2,3']bipyridinyl is further characterized by the differential scanning calorimetry (DSC) thermograph shown in Figure 26, which shows an endothermic event corresponding to a melt with an onset temperature of approximately 194 °C. The melting point is in the range 206 to 208 °C.

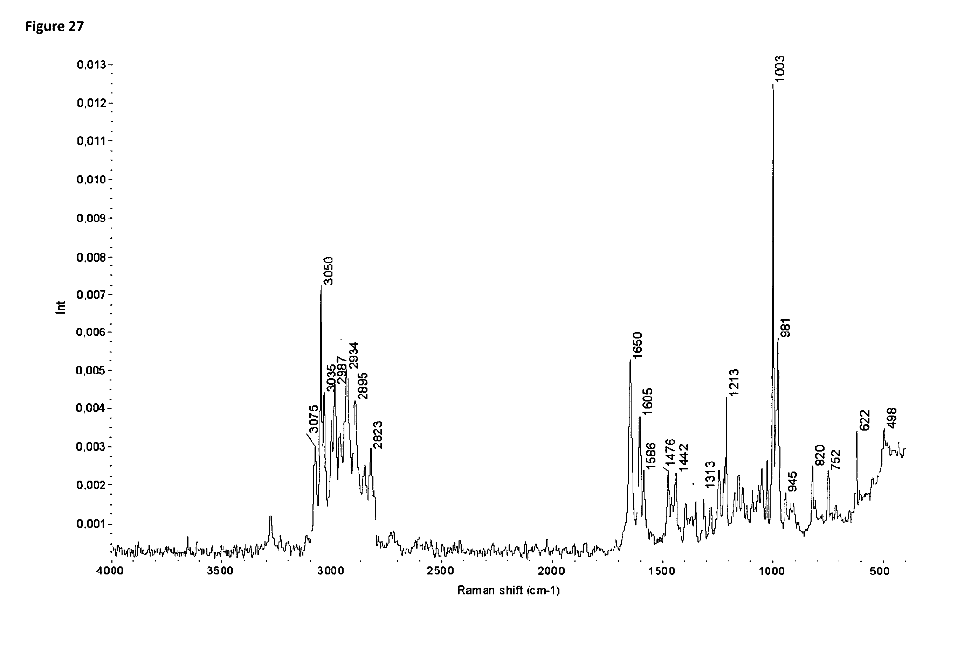

This saccharin salt of 5-chloro-3-(4-methanesulfonylphenyl)-6'-methyl-[2,3']bipyridinyl is also characterized by the Raman spectrum given in Figure 27, which shows characteristic absorptions at 3058, 2915, 1643, 1597, 1574, 1431, 1367 cm⁻¹.

Another embodiment of the invention is the hydrochloride salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 8.1, 15.1, 16.3, 16.8, 19.0, 20.5, 21.3, 21.9, 24.5, 24.9, 25.6, 26.1 and 29.3° ± 0.5°.

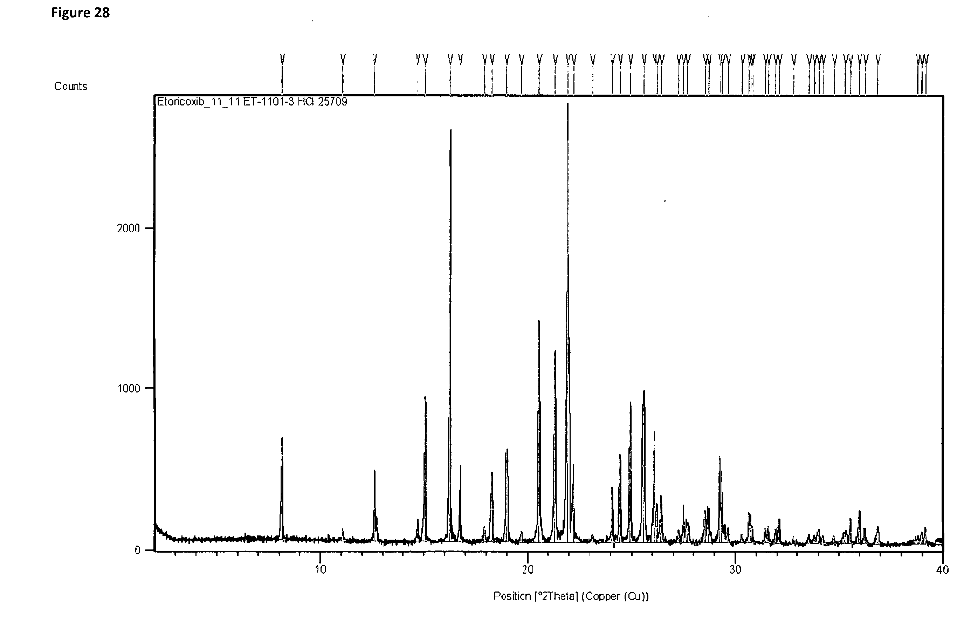

The XRPD pattern is given in Figure 28 with the main peaks listed in Table 10.

**Table 10 Table of diffraction peaks for the hydrochloride salt of 5-chloro-3-(4-methanesulfonylphenyl)-6'-methyl-[2,3']bipyridinyl.**

| Pos. [°2Th.] | d-spacing [A]=0.1nm | Rel. Int. [%] |
|---|---|---|
| 8.13 | 10.863 | 23.2 |
| 12.61 | 7.012 | 16.1 |
| 15.05 | 5.881 | 35.3 |
| 16.25 | 5.450 | 93.4 |
| 16.75 | 5.289 | 20.8 |
| 18.25 | 4.856 | 16.1 |
| 19.00 | 4.668 | 23.0 |
| 20.53 | 4.322 | 54.9 |
| 21.32 | 4.164 | 46.1 |
| 21.92 | 4.052 | 100.0 |
| 24.09 | 3.692 | 13.3 |
| 24.45 | 3.638 | 20.7 |
| 24.92 | 3.570 | 32.1 |
| 25.57 | 3.480 | 38.0 |
| 26.08 | 3.413 | 25.4 |
| 29.25 | 3.051 | 20.4 |
| 30.68 | 2.912 | 7.0 |
| 35.98 | 2.494 | 6.3 |

Another embodiment of the invention is the hydrobromide salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 14.7, 16.1, 18.0, 20.3, 21.1, 21.6, 24.7, 25.5 and 28.8° ± 0.5°.

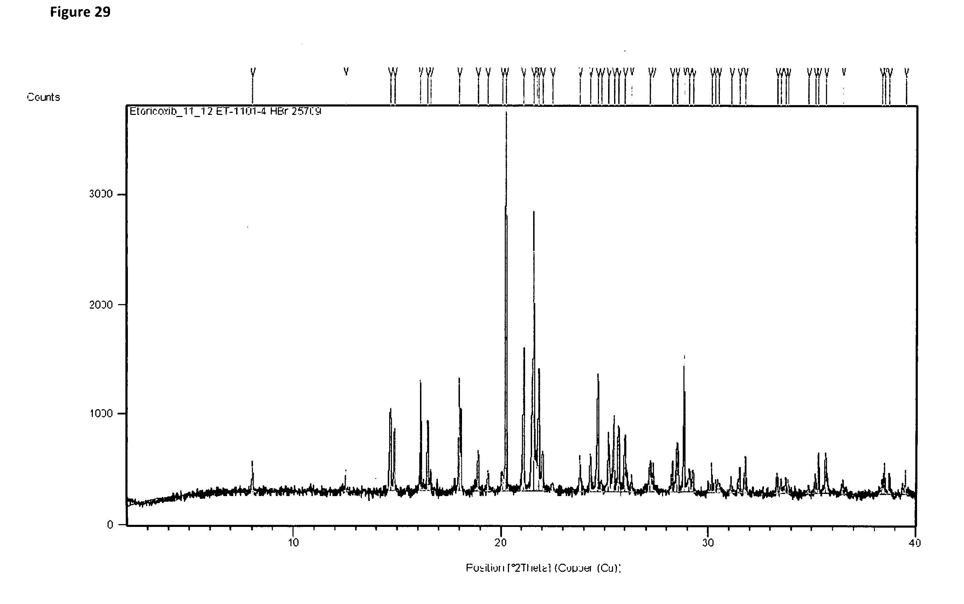

The XRPD pattern is given in Figure 29 with the main peaks listed in Table 8.

**Table 11 Table of diffraction peaks for the hydrobromide salt of 5-chloro-3-(4-methanesulfonylphenyl)-6'-methyl-[2,3']bipyridinyl.**

| Pos. [°2Th.] | d-spacing [A]=0.1nm | Rel. Int. [%] |
|---|---|---|
| 8.05 | 10.970 | 7.0 |
| 14.70 | 6.022 | 21.7 |
| 16.14 | 5.487 | 28.7 |
| 16.48 | 5.376 | 16.9 |
| 18.01 | 4.921 | 28.3 |
| 20.26 | 4.379 | 100.0 |
| 21.09 | 4.210 | 37.9 |
| 21.58 | 4.114 | 63.5 |
| 24.66 | 3.608 | 30.1 |
| 25.18 | 3.534 | 13,9 |
| 25.45 | 3.497 | 19,4 |
| 26.00 | 3.425 | 13,4 |
| 27.20 | 3.276 | 7,3 |
| 28.50 | 3.129 | 12,8 |
| 28.82 | 3.095 | 35,3 |
| 30.16 | 2.961 | 6,9 |
| 31.77 | 2.815 | 9,1 |
| 35.30 | 2.540 | 10,2 |
| 35.66 | 2.516 | 9,7 |

Another embodiment of the invention is the nitrate salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 16.3, 16.7, 17.4, 19.3, 22.4, 23.3, 26.5, 29.2 and 30.4 ° ± 0.5°.

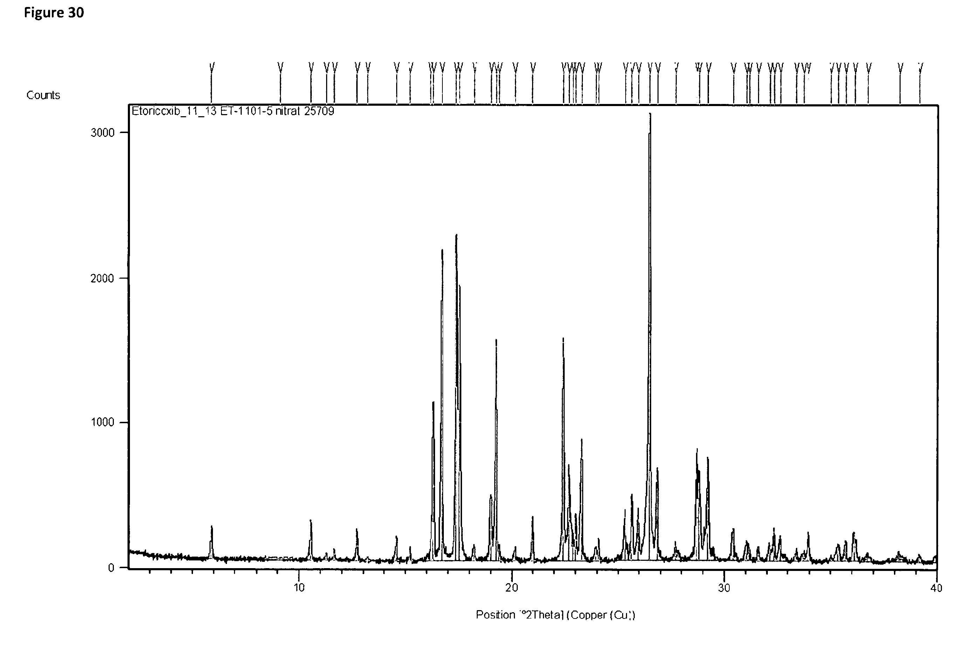

The XRPD pattern is given in Figure 30 with the main peaks listed in Table 12.

**Table 12 Table of diffraction peaks for the nitrate salt of 5-chloro-3-(4-methanesulfonylphenyl)-6'-methyl-[2,3']bipyridinyl.**

| Pos. [°2Th.] | d-spacing [A]=0.1nm | Rel. Int. [%] |
|---|---|---|
| 5.91 | 14.949 | 6.8 |
| 10.54 | 8.385 | 8.7 |
| 12.72 | 6.955 | 6.5 |
| 16.32 | 5.426 | 33.5 |
| 16.71 | 5.301 | 67.5 |
| 17.40 | 5.091 | 68.9 |
| 19.25 | 4.608 | 47.9 |
| 22.42 | 3.962 | 49.0 |
| 22.70 | 3.914 | 18.8 |
| 23.26 | 3.821 | 25.6 |
| 25.66 | 3.470 | 13.9 |
| 26.45 | 3.367 | 100.0 |
| 28.69 | 3.109 | 19.5 |
| 29.22 | 3.054 | 23.3 |
| 30.39 | 2.939 | 7.0 |
| 32.32 | 2.768 | 6.7 |
| 33.94 | 2.639 | 6.0 |
| 36.12 | 2.485 | 6.2 |

Another embodiment of the invention is the sulfate salt of 5-chloro-3-(4-methanesulfonylphenyl)-6'-methyl-[2,3']bipyridinyl, having the empirical formula (C15 H17 N 02, C4 H4 04) and characterized by an XRPD pattern with major peaks at the following 2-theta values measured using CuKα radiation: 10.2, 12.3, 16.2, 16.5, 17.3, 18.0, 20.3, 22.9, 24.3, 26.9 and 30.5° ± 0.5°.

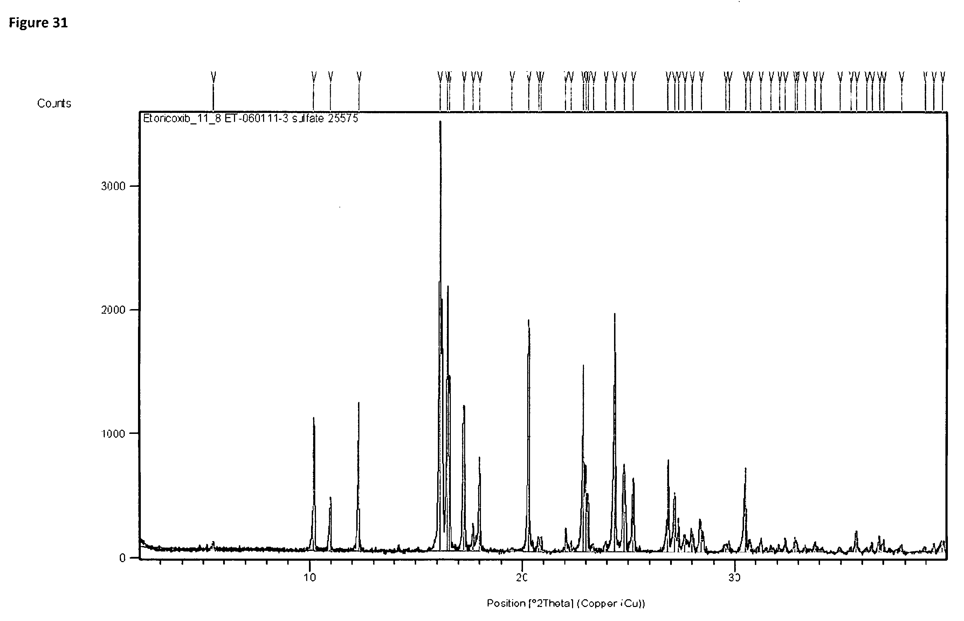

The XRPD pattern is given in Figure 31 with the main peaks listed in Table 13.

**Table 13 Table of diffraction peaks for the sulfate salt of 5-chloro-3-(4-methanesulfonylphenyl)-6'-methyl-[2,3']bipyridinyl.**

| Pos. [°2Th.] | d-spacing [A]=0.1nm | Rel. Int. [%] |
|---|---|---|
| 10.20 | 8.661 | 29.0 |
| 10.97 | 8.060 | 12.1 |
| 12.33 | 7.175 | 34.0 |
| 16.15 | 5.483 | 100.0 |
| 16.49 | 5.371 | 63.4 |
| 17.26 | 5.133 | 34.4 |
| 18.00 | 4.923 | 21.7 |
| 20.31 | 4.369 | 55.5 |
| 22.86 | 3.887 | 46.0 |
| 24.34 | 3.654 | 56.5 |
| 24.81 | 3.585 | 19.1 |
| 25.22 | 3.528 | 17.3 |
| 26.87 | 3.316 | 21.2 |
| 27.16 | 3.281 | 14.1 |
| 28.43 | 3.137 | 6.5 |
| 30.48 | 2.930 | 19.3 |
| 35.73 | 2.511 | 5.1 |

When it is stated that the present invention relates to a crystalline form of Etoricoxib the degree of crystallinity is preferably greater than about 60%. More preferably it is greater than about 80%. It is particularly preferable if the degree of crystallinity is greater than about 90%. It is more particularly preferable if the degree of crystallinity is greater than 95%. It is most particularly preferable if the degree of crystallinity is greater than about 98%.

It is known by those ordinarily skilled in the art that X-ray powder diffraction patterns may be obtained that have one or more measurement errors depending on the measurement conditions, including but not limited to the instrument model or specific diffractometer used. In particular, it is generally known that the intensities in an X-ray powder diffraction pattern may fluctuate depending on the measurement conditions. Therefore it should be understood that the etoricoxib salts of the present invention are not limited to crystals that provide X-ray powder diffraction patterns identical to the X-ray powder diffraction patterns shown here and any crystals providing X-ray powder diffraction patterns substantially the same as those shown in herein fall within the scope of the present invention. A person skilled in the art of X-ray powder diffraction is able to judge the substantial identity of X-ray powder diffraction patterns.

Persons skilled in the art of X-ray powder diffraction will be aware of the fact that the relative intensities of peaks can be affected by several factors. These include samples with a grain size larger than 30 microns or non-unitary aspect ratios that may affect the analysis of the samples. The skilled person will also know that the positions of the reflections can be affected by the precise height at which the sample is mounted in the diffractometer and the zero calibration of the diffractometer. The surface planarity of the sample may also have an effect. For these reasons the pattern diffraction data presented are not to be construed as absolute values.

Generally the measurement error in the diffraction angle of a peak in an X-ray powder diffractogram is approximately 5% or less, in particular plus or minus 0.5° 2-theta and this degree of measurement should be taken into account when considering the X-ray diffraction pattern given in the figures and when reading the tables. It must further be understood that factors relating to experimental conditions and sample preparation such as preferred orientation will cause fluctuations in the relative intensities of peaks in the diffraction pattern.

XRPD patterns were measured with laboratory X-Ray diffractometer X'PERT PRO MPD PANalytical operating in diffraction mode θ - θ with copper radiation CuKα (λ=1.542 Å, 45 kV/ 40 mA), graphite monochromator, in 2theta range 2 - 40° 2θ, with step size 0.01° 2θ and time per step 50 s. Primary optics setting: Soller slits 0.02 rad, automatic PDS, 10 mm mask, 1/4° anti-scatter slit, irradiated sample area 10 mm. Secondary optics setting: 5.0 mm anti-scatter slit, Soller slits 0.02 rad, detector X'Celerator with maximal active length. Samples were measured on silica plate holder.

All the DSC thermographs reported herein were acquired with a scanning rate of 10 °C/min.

### List of Drawings

**Figure 1****:** XRPD pattern of the succinic acid salt of 5-chloro-3-(4-methanesulfonylphenyl)-6'-methyl-[2,3']bipyridinyl.
**Figure 2****:** DSC thermograph of the succinic acid salt of 5-chloro-3-(4-methanesulfonylphenyl)-6'-methyl-[2,3']bipyridinyl.
**Figure 3****:** Raman spectrum of the succinic acid salt of 5-chloro-3-(4-methanesulfonylphenyl)-6'-methyl-[2,3']bipyridinyl.
**Figure 4****:** DVS isotherm plot of the succinic acid salt of 5-chloro-3-(4-methanesulfonylphenyl)-6'-methyl-[2,3']bipyridinyl.
**Figure 5****:** XRPD pattern of the adipic acid salt of 5-chloro-3-(4-methanesulfonylphenyl)-6'-methyl-[2,3']bipyridinyl.
**Figure 6****:** DSC thermograph of the adipic acid salt of 5-chloro-3-(4-methanesulfonylphenyl)-6'-methyl-[2,3']bipyridinyl.
**Figure 7****:** Raman spectrum of the adipic acid salt of 5-chloro-3-(4-methanesulfonylphenyl)-6'-methyl-[2,3']bipyridinyl.
**Figure 8****:** XRPD pattern of Form A of the fumaric acid salt of 5-chloro-3-(4-methanesulfonylphenyl)-6'-methyl-[2,3']bipyridinyl.
**Figure 9****:** DSC thermograph of Form A of the fumaric acid salt of 5-chloro-3-(4-methanesulfonylphenyl)-6'-methyl-[2,3']bipyridinyl.
**Figure 10****:** Raman spectrum of Form A of the fumaric acid salt of 5-chloro-3-(4-methanesulfonylphenyl)-6'-methyl-[2,3']bipyridinyl.
**Figure 11****:** DVS isotherm plot of Form A of the fumaric acid salt of 5-chloro-3-(4-methanesulfonylphenyl)-6'-methyl-[2,3']bipyridinyl.
**Figure 12****:** XRPD pattern of Form B of the fumaric acid salt of 5-chloro-3-(4-methanesulfonylphenyl)-6'-methyl-[2,3']bipyridinyl.
**Figure 13****:** DSC thermograph of Form B of the fumaric acid salt of 5-chloro-3-(4-methanesulfonylphenyl)-6'-methyl-[2,3']bipyridinyl.
**Figure 14****:** XRPD pattern of the benzoic acid salt of 5-chloro-3-(4-methanesulfonylphenyl)-6'-methyl-[2,3']bipyridinyl.
**Figure 15****:** DSC thermograph of the benzoic acid salt of 5-chloro-3-(4-methanesulfonylphenyl)-6'-methyl-[2,3']bipyridinyl.
**Figure 16****:** Raman spectrum of the benzoic acid salt of 5-chloro-3-(4-methanesulfonylphenyl)-6'-methyl-[2,3']bipyridinyl.
**Figure 17****:** XRPD pattern of the salicylic acid salt of 5-chloro-3-(4-methanesulfonylphenyl)-6'-methyl-[2,3']bipyridinyl.
**Figure 18****:** DSC thermograph of the salicylic acid salt of 5-chloro-3-(4-methanesulfonylphenyl)-6'-methyl-[2,3']bipyridinyl.
**Figure 19****:** Raman spectrum of the salicylic acid salt of 5-chloro-3-(4-methanesulfonylphenyl)-6'-methyl-[2,3']bipyridinyl.
**Figure 20****:** XRPD pattern of the 1-hydroxy-2-naphthoic acid salt of 5-chloro-3-(4-methanesulfonylphenyl)-6'-methyl-[2,3']bipyridinyl.
**Figure 21****:** DSC thermograph of the 1-hydroxy-2-naphthoic acid salt of 5-chloro-3-(4-methanesulfonylphenyl)-6'-methyl-[2,3']bipyridinyl.
**Figure 22****:** Raman spectrum of the 1-hydroxy-2-naphthoic acid salt of 5-chloro-3-(4-methanesulfonylphenyl)-6'-methyl-[2,3']bipyridinyl.
**Figure 23****:** XRPD pattern of the 3-hydroxy-2-naphthoic acid salt of 5-chloro-3-(4-methanesulfonylphenyl)-6'-methyl-[2,3']bipyridinyl.
**Figure 24****:** DSC thermograph of the 3-hydroxy-2-naphthoic acid salt of 5-chloro-3-(4-methanesulfonylphenyl)-6'-methyl-[2,3']bipyridinyl.
**Figure 25****:** Raman spectrum of the 3-hydroxy-2-naphthoic acid salt of 5-chloro-3-(4-methanesulfonylphenyl)-6'-methyl-[2,3']bipyridinyl.
**Figure 26****:** XRPD pattern of the saccharin salt of 5-chloro-3-(4-methanesulfonylphenyl)-6'-methyl-[2,3']bipyridinyl.
**Figure 27****:** DSC thermograph of the saccharin salt of 5-chloro-3-(4-methanesulfonylphenyl)-6'-methyl-[2,3']bipyridinyl.
**Figure 28****:** Raman spectrum of the saccharin salt of 5-chloro-3-(4-methanesulfonylphenyl)-6'-methyl-[2,3']bipyridinyl.
**Figure 29****:** XRPD pattern of the hydrochloride salt of 5-chloro-3-(4-methanesulfonylphenyl)-6'-methyl-[2,3']bipyridinyl.
**Figure 30****:** XRPD pattern of the hydrobromide salt of 5-chloro-3-(4-methanesulfonylphenyl)-6'-methyl-[2,3']bipyridinyl.
**Figure 31****:** XRPD pattern of the nitrate salt of 5-chloro-3-(4-methanesulfonylphenyl)-6'-methyl-[2,3']bipyridinyl.
**Figure 32:** XRPD pattern of the sulfate salt of 5-chloro-3-(4-methanesulfonylphenyl)-6'-methyl-[2,3']bipyridinyl.

### Examples

### Example 1: General method for the preparation of 5-chloro-3-(4-methanesulfonylphenyl)-6'-methyl-[2,3']bipyridinyl salts by evaporation

500mg of N5-chloro-3-(4-methanesulfonylphenyl)-6'-methyl-[2,3']bipyridinyl was dissolved in 5ml of isopropanol or acetone at 45°C and one stoichiometric equivalent of the counterion was added as a solution in methanol. The solution was heated to a temperature between ambient and reflux to ensure complete dissolution and the solvent was allowed to evaporate at room temperature to crystallize the 5-chloro-3-(4-methanesulfonylphenyl)-6'-methyl-[2,3']bipyridinyl salts.

### Example 2: Preparation of the succinic acid salt of 5-chloro-3-(4-methanesulfonylphenyl)-6'-methyl-[2,3']bipyridinyl.

10.0g of 5-chloro-3-(4-methanesulfonylphenyl)-6'-methyl-[2,3']bipyridinyl was dissolved in 50ml of isopropanol at 80°C to give a slightly yellow solution . 3.3g of succinic acid was added to the solution as a solid and dissolved, the solution was stirred for 15 minutes then cooled. A white solid precipitated from the solution at 30°C to give a thick slurry. The solid was isolated by filtration and dried in a vacuum oven for a final yield of 10.8g of the succinic acid salt (81%).

### Example 3: Preparation of the adipic acid salt of 5-chloro-3-(4-methanesulfonylphenyl)-6'-methyl-[2,3']bipyridinyl

250mg 5-chloro-3-(4-methanesulfonylphenyl)-6'-methyl-[2,3']bipyridinyl and 102 mg of adipic acid were dissolved in 15ml of isopropanol at 65°C to give a slightly yellow solution. The solution was cooled to room temperature then allowed to evaporate to yield the desired adipic acid salt as a white powder.

### Example 4: Preparation of Form A of the fumaric acid salt of 5-chloro-3-(4-methanesulfonylphenyl)-6'-methyl-[2,3']bipyridinyl.

10.0g of 5-chloro-3-(4-methanesulfonylphenyl)-6'-methyl-[2,3']bipyridinyl was dissolved in 50ml of isopropanol at 80°C to give a slightly yellow solution. 3.3g of fumaric acid was added to the solution as a solid and dissolved, the solution was stirred for 5 minutes then cooled. A white solid precipitated from the solution at 30°C to give a thick slurry. The slurry was cooled to 15 °C and the solid was isolated by filtration and dried in a vacuum oven for a final yield of 10.3 g of the fumaric acid salt (78%).

### Example 5: Preparation of the saccharin salt of 5-chloro-3-(4-methanesulfonylphenyl)-6'-methyl-[2,3']bipyridinyl.

1.0g of 5-chloro-3-(4-methanesulfonylphenyl)-6'-methyl-[2,3']bipyridinyl and 0.5g of saccharin were dissolved in 20ml of acetone at 40 °C. The solution was cooled to 5 °C to crystallize the desired saccharin salt.

### Example 6: Method for assessing polymorphic stability of the succinic acid salt and Form A of the fumaric acid salt of 5-chloro-3-(4-methanesulfonylphenyl)-6'-methyl-[2,3']bipyridinyl.

Approximately 50-100 mg of the salts under investigation were suspended in 1ml of water, ethanol, 1-propanol, 2-propanol, 1-butanol, 2-butanol, tert-butanol, 1-pentanol, acetone, methyl ethyl ketone, isobutyl methyl ketone, ethyl formate, methyl acetate, ethyl acetate, propyl acetate, isopropyl acetate, butyl acetate, isobutyl acetate, tetrahydrofuran, 1,4-dioxane, toluene, acetonitrile and N-methyl pyrrolidine. The suspensions were agitated at a temperature of 25°C for 35 days. The excess solvent was removed and the samples evaporated to dryness in a vacuum oven at 25 °C.

## Claims

1. Crystalline salt of 5-chloro-3-(4-methanesulfonylphenyl)-6'-methyl-[2,3']bipyridinyl with an organic acid selected from succinic, adipic, fumaric, benzoic, salicylic, 1-hydroxy-2-naphthoic, 3-hydroxy-2-naphthoic acid and saccharin and/or an inorganic acid selected from hydrochloric, hydrobromic, nitric, sulfuric acid.

2. A succinic salt according to claim 1, wherein the solid state form is **characterised by** an XRPD pattern with major peaks at the following 2-theta values measured using CuKα radiation: 6.4, 16.3, 17.9, 18.2, 19.6, 20.9, 22.3, 25.6 and 30.7° ± 0.5°.

3. An adipic salt according to claim 1, wherein the solid state form is **characterised by** an XRPD pattern with major peaks at the following 2-theta values measured using CuKα radiation: 6.0, 7.5, 14.0, 17.2, 17.5, 19.7, 20.2, 21.0, 21.4, 24.4 and 28.8° **±** 0.5°.

4. A fumaric salt A according to claim 1, wherein the solid state form is **characterised by** an XRPD pattern with major peaks at the following 2-theta values measured using CuKα radiation: 11.1, 12.8, 15.7, 16.2, 17.5, 18.0, 20.8, 21.6, 22.3, 23.2, 24.0, 25.6, 26.4 and 26.8° ± 0.5°.

5. A fumaric salt B according to claim 1, wherein the solid state form is **characterised by** an XRPD pattern with major peaks at the following 2-theta values measured using CuKα radiation: 6.39, 16.2, 18.3, 20.9, 24.6, 25.8, 31.0° ± 0.5°.

6. A benzoic salt according to claim 1, wherein the solid state form is **characterised by** an XRPD pattern with major peaks at the following 2-theta values measured using CuKα radiation: 9.6, 15.4, 19.1, 20.6, 22.0, 22.3 and 27.9° ± 0.5°.

7. A salicylic salt according to claim 1, wherein the solid state form is **characterised by** an XRPD pattern with major peaks at the following 2-theta values measured using CuKα radiation: 8.9, 16.8, 17.1, 17.8, 20.4, 24.4, 26.8, 27.2° ± 0.5°.

8. A 1-hydroxy-2-naphthoic salt according to claim 1, wherein the solid state form is **characterised by** an XRPD pattern with major peaks at the following 2-theta values measured using CuKα radiation: 7.4, 14.9, 15.2, 16.7, 18.8, 21.5, 21.8, 22.8, 24.0 and 29.6° ± 0.5°.

9. A 3-hydroxy-2-naphthoic salt according to claim 1, wherein the solid state form is **characterised by** an XRPD pattern with major peaks at the following 2-theta values measured using CuKα radiation: 6.7, 10.1, 15.3, 15.5, 17.4, 19.0, 20.1, 20.5, 21.3, 24.6 and 27.3° ± 0.5°.

10. A saccharin salt according to claim 1, wherein the solid state form is **characterised by** an XRPD pattern with major peaks at the following 2-theta values measured using CuKα radiation: 6.1, 12.2, 12.7, 15.3, 16.5, 17.7, 18.4, 19.7, 20.6, 20.9, 21.4, 22.4, 23.5, 24.0, 24.6, 25.6, 26.0, 26.4, 29.1° ± 0.5°.

11. A hydrochloride salt according to claim 1, wherein the solid state form is **characterised by** an XRPD pattern with major peaks at the following 2-theta values measured using CuKα radiation: 8.1, 15.1, 16.3, 16.8, 19.0, 20.5, 21.3, 21.9, 24.5, 24.9, 25.6, 26.1 and 29.3° ± 0.5°.

12. A hydrobromide salt according to claim 1, wherein the solid state form is **characterised by** an XRPD pattern with major peaks at the following 2-theta values measured using CuKα radiation: 14.7, 16.1, 18.0, 20.3, 21.1, 21.6, 24.7, 25.5 and 28.8° ± 0.5°.

13. A nitrate salt according to claim 1, wherein the solid state form is **characterised by** an XRPD pattern with major peaks at the following 2-theta values measured using CuKα radiation: 16.3, 16.7, 17.4, 19.3, 22.4, 23.3, 26.5, 29.2 and 30.4° ± 0.5°.

14. A sulfate salt according to claim 1, wherein the solid state form is **characterised by** an XRPD pattern with major peaks at the following 2-theta values measured using CuKα radiation: 10.2, 12.3, 16.2, 16.5, 17.3, 18.0, 20.3, 22.9, 24.3, 26.9 and 30.5° ± 0.5°.

15. A process for preparing any of the salts or cocrystals defined in Claims 1-14, which comprises dissolving 5-chloro-3-(4-methanesulfonylphenyl)-6'-methyl-[2,3']bipyridinyl in a suitable organic solvent or mixture of solvents such as acetone, methanol, tetrahydrofuran, ethyl acetate or water at a temperature between 20°C and reflux temperature and adding a stoichiometric amount of the salt or cocrystal former dissolved in a suitable organic solvent or mixture of solvents such as methanol, ethanol or water and crystallizing the salt by cooling the solution to a temperature between 20°C and 30°C and allowing the solvent to evaporate.

16. A process for preparing any of the salts defined in claims 1-11, which comprises dissolving 5-chloro-3-(4-methanesulfonylphenyl)-6'-methyl-[2,3']bipyridinyl and a stoichiometric amount of the salt former in a suitable solvent such as methanol, tetrahydrofuran, ethyl acetate or isopropylalcohol at a temperature between 20°C and the boiling point of the solvent and cooling the solution to a temperature between 0°C and 30°C and holding at this temperature for 1 to 7 days to crystallize the salt.

17. A process for preparing any of the salts of claims 1-3, which comprises dissolving 5-chloro-3-(4-methanesulfonylphenyl)-6'-methyl-[2,3']bipyridinyl and one molar equivalent of the salt former in the minimum volume of a suitable solvent by heating to a temperature between 30°C and the boiling point of the solvent and adding further solvent until complete dissolution of the solid is observed, then cooling the solution to a temperature between the boiling point of the solvent and ambient temperature to crystallize the desired salt form, which is then isolated by filtration.

18. A process for the preparation of any of the salts of claims 8 -11, which comprises dissolving 5-chloro-3-(4-methanesulfonylphenyl)-6'-methyl-[2,3']bipyridinyl in a suitable solvent at a temperature between room temperature and 50°C and adding one molar equivalent of the salt former dissolved in an appropriate solvent to precipitate the salt, which is isolated by filtration.
